# EUROPEAN PATENT SPECIFICATION

(11) **EP 2 490 729 B1**
(45) Date of publication and mention of the grant of the patent: **31.03.2021**
(21) Application number: 10825753.6
(22) Date of filing: 22.10.2010
(51) Int. Cl.: A61M 1/10, A61M 1/12, A61F 2/06

(54) **INTRA-AORTIC BALLOON PUMP AND DRIVER**
INTRAAORTALE BALLONPUMPE UND ANTRIEBSVORRICHTUNG DAFÜR
POMPE DE BALLON DE CONTRE-PULSION INTRA-AORTIQUE ET DISPOSITIF DE COMMANDE

(30) Priority: 22.10.2009 US 604228
(43) Date of publication of application: 29.08.2012
(73) Proprietor: NuPulseCV, Inc., Tuscon, AZ 85718 (US)
(72) Inventor: JEEVANANDAM, Valluvan, New York, NY 10011 (US); SNYDER, Roger, William, New York, NY 10011 (US); SMITH, Robert, New York, NY 10011 (US); DEDECKER, Paul, New York, NY 10011 (US)
(74) Representative: Forresters IP LLP
(86) International application number: PCT/US2010/053779
(87) International publication number: WO 2011/050279

(56) References cited:
- WO-A1-01/05446
- JP-A- 2002 518 106
- JP-A- 2005 000 434
- US-A- 4 634 430
- US-B1- 6 468 200
- US-B1- 7 374 531

## Description

### SUMMARY

Devices and methods are disclosed for implanting, positioning, removing, replacing and operating intra-aortic balloon pumps.

### BACKGROUND

The use of intraaortic balloon pumps is a well known method for treating heart failure. The balloon pump is positioned inside the aorta, typically in the proximal descending aorta. The balloon pump (typically 40-50 milliliters in capacity) is inflated and deflated in time with the contraction of the left ventricle. During diastole, the balloon is inflated, thereby driving blood in the ascending aorta and aortic arch into the coronary arteries to supply oxygen to the heart muscle. During systole, as the left ventricle contracts, the balloon is deflated so as to decrease the afterload. This procedure is termed "counterpulsation."

Such balloon pumps, for example PCT publication No. WO 01/05446 (Datascope Investment Corp), also typically require burdensome external equipment to operate, such as gas compressors, gas tanks, and/or condensors.

### BRIEF DESCRIPTION OF THE DRAWINGS

**FIG. 1** schematically shows an intraaortic balloon pump implanted in a patient using an arterial interface.
**FIG. 2** schematically shows an intraaortic balloon pump positioned in the proximal descending aorta, with the pump's inflation catheter entering the vasculature at the right subclavian artery through an arterial interface.
**FIG. 3** schematically shows a ventricular assist device including an intra-aortic balloon pump, an internal drive line, a skin interface, an external drive line, and an external driver.
**FIG. 4** schematically shows a driver for a ventricular assist device including a bellows and multiple valves.
**FIG. 5** schematically shows a mover for operating a bellows.
**FIG. 6** schematically shows a skin interface.
**FIGS. 7** and **7A** schematically show balloon pump assemblies including a drive line with regions of varying diameter.
**FIGS. 8A** and **8B** show two views of an embodiment of a patient connector.
**FIG. 9** is a flowchart describing a method of leak detection.
**FIG. 10** is a flowchart describing a method of controlling the time needed for inflation of the balloon pump.
**FIG. 11** is a flowchart describing a method of initializing the system.
**FIG. 12** is a flowchart describing a method of using an EKG signal to detect the QRS complex.
**FIG. 13** is a flowchart describing a method of detecting a dicrotic notch.
**FIG. 14** is a flowchart describing a method of operating the system in closed mode.
**FIG. 15** is a flowchart describing a method of operating the system in open mode.
**FIG. 16** is a flowchart describing a method of purging and filling the system with ambient air.

### DETAILED DESCRIPTION

Existing ventricular assist devices and intraaortic balloon pumps suffer from the problem of using inconvenient external apparatuses. Many intraaortic balloon pump systems use helium as a pumping medium, which requires that the patient connect to a cumbersome helium tank. Helium is chosen largely for its extremely low viscosity, allowing the use of a relatively thin drive line between the balloon pump and an external driver. (According to Poiseuille's law, the pressure drop along a fluid line is proportional to the fluid viscosity and inversely proportional to the fourth power of the diameter. A sufficiently low-viscosity fluid can therefore reduce the pressure drop to acceptable levels along even a thin line.) The thin internal drive line is in turn required to avoid occluding the artery through which the drive line is threaded.

Rather than resort to using low-viscosity helium and the attendant helium tank, the inventors have taken a different approach by minimizing the length of the portion of the drive line that must be especially thin. Some parts of the drive line may pass through the thoracic and/or abdominal cavities; there is no reason to keep this section of the line especially thin. Likewise, the portion of the drive line actually dangling in the aorta need not be particularly thin since the aorta is large and is in relatively little danger of being occluded by the drive line. The inventors realized that a small diameter is of greatest benefit in the part of the drive line deployed in a lesser artery, such as the right or left subclavian arteries, the common carotid arteries, or the brachiocephalic (innominate) artery. By keeping the drive line relatively wide in most of its length and narrow only where absolutely necessary, ordinary air can be used as the pumping medium, eliminating the need for a helium tank. Some devices and methods disclosed herein result from this insight.

Existing intraaortic balloon pumps can also be cumbersome because many require removal of humidity from the pumping medium. In essentially any system that causes a pumping medium to interact with blood through anything less than a perfectly water-impermeable material, moisture will gradually seep through the balloon pump and/or drive lines, contaminating the pumping medium with water vapor. Humidity in the pumping medium can change the fluid mechanics of pumping and also increase the risk of microbial contamination. Where the pumping medium is helium, the medium must be conserved while the water is removed, hence the need for a compressor.

Because the inventors have eliminated the need for helium, a far simpler solution is possible, namely external venting of the pumping medium. Using air instead of helium as a pumping medium means that there is always an infinite supply of pumping medium on hand. When the air in the pump has become too moist, one can simply purge the air from the device and fill the device with relatively dry ambient air. In some embodiments described below, an external driver can continuously shift from operating the pump in a closed mode, in which the system is sealed so that no air enters or leaves the system, to an open mode, in which the system can operate without interruption while replacing the air already in the system with fresh external air. The air in the system can be replaced at regular intervals, or only when triggered, for instance by a humidity sensor.

A much more portable system results from eliminating the need for both a helium tank and compressor.

A system that can operate in a closed mode as described above has the added benefit of leak detection. Because no air should be entering or leaving the system in closed mode, the pressure in the system should be the same at identical points in the pumping cycle. If the pressure at a given point in the cycle is dropping over time, one can be confident that there is an air leak somewhere in the system, information that is important to communicate to the patient or a physician.

The inflation/deflation cycles can be triggered based on QRS complex detection from electrocardiogram (EKG) data, by dicrotic notch detection from pressure data, or by both. Electrodes and pressure sensors can be provided as necessary. The balloon itself may function as a pressure sensor, especially in a partially deflated state. Deflation will typically be triggered based on the detection of a QRS complex, which indicates impending systole, while inflation will typically be triggered based on the detection of a dicrotic notch, which indicates the beginning of diastole. Both inflation and deflation events can be triggered by one set of data; for example, inflation may be triggered at a certain predetermined amount of time after QRS detection.

Another problem in using existing intraaortic balloon pumps as long-term devices is that parts can wear out, cause infections, or otherwise need to be replaced. After the graft is attached at the incision in the artery and thereby exposed to the bloodstream, the healing process causes clotted blood, granulation tissue and other material to accumulate around the incision and in the graft. Such tissue completely fills the available volume inside the graft except for the space occupied by the inflation catheter. Such tissue becomes a cohesive, sometimes solid, mass with the graft. Because the balloon, even in its deflated state, is much larger than the inflation catheter (the catheter being small to avoid occupying too much cross-section of the vasculature through which it runs), it is practically impossible to remove the balloon through the clogged graft or to thread a new balloon through. The current solution to this problem is to replace the entire graft every time the balloon is replaced, which requires repeating the highly invasive vascular grafting procedure from the beginning.

The focus, then, has been on avoiding failures that necessitate the costly and dangerous replacement surgeries. For example, extreme care is taken to avoid introducing infections, despite inconvenience and discomfort to the patient. Also, the pumps are made of especially durable materials that are resistant to normal body stresses, even at the expense of more desirable functional characteristics.

But the inventors realized that failures are inevitable; practically no implantable device can forever survive the stresses placed upon it by the living body. Living tissue is constantly repaired and maintained by normal body processes, while implanted devices tend to be attacked, compartmentalized, or otherwise isolated. At the very least, they do not benefit from normal repair and maintenance processes to help them resist normal stresses.

So the inventors hit upon an entirely new strategy: rather than continue dogged efforts at finding ways to prevent failures, they accepted that failures cannot be avoided and instead sought ways to make the replacement procedure faster, simpler, and safer. Some disclosed systems and methods for interfacing the intraaortic balloon pump with the vasculature resulted from this strategy.

The scope of the present invention is defined by the appended claims.

The vascular interface incorporates a "stopper" to fill the space between the graft and the inflation catheter. Because this space is filled from the beginning, body processes cannot invade the graft to fill that space with clotted blood, etc. (although there may be some minimal invasion around the stopper itself). As a result, when the time to replace the pump inevitably comes, the stopper can be slipped out of the graft, leaving a largely patent graft lumen. The graft lumen is wide enough to permit removal and replacement of the pump. The graft itself need not be removed and replaced, so the dangerous and time-consuming step of vascular surgery is avoided.

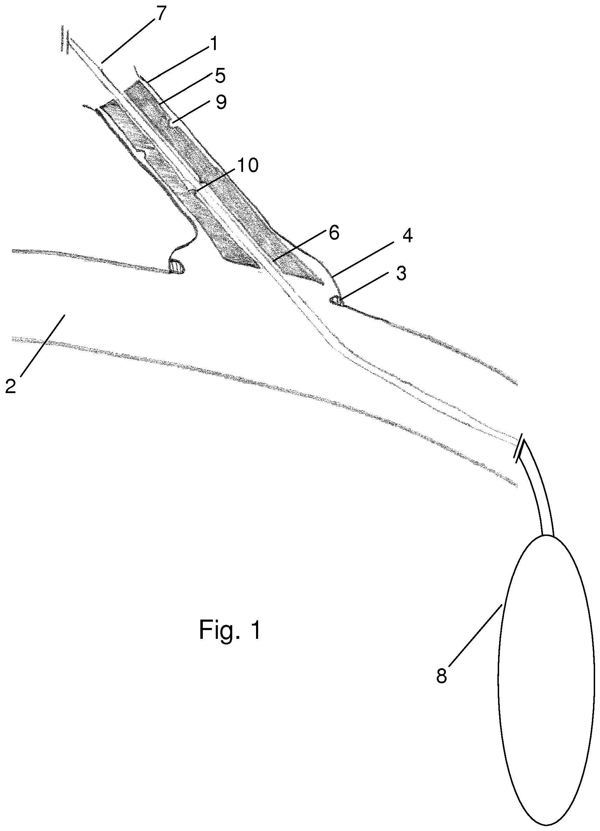

**FIG. 1** schematically shows an example of such a device, as deployed in a patient's vasculature. A vascular graft **1** is attached to an artery **2** with a suture ring **3** at the position of an incision in the artery. The particular graft shown flares at its distal end **4.** The stopper **5** sits inside the graft **1**, filling the interior of the graft **1** except for a hole **6** along the length of the stopper **5.** The hole **6** necessarily runs the entire length of the stopper **5**, but the stopper **5** need not run the entire length of the graft **1.** It is sufficient that some part of the stopper **5** is near the distal end of the graft **4** when properly positioned. In some cases, the stopper can extend out past the proximal end of the graft, to help minimize clot invasion. The stopper can be secured to and immobilized with respect to the graft.

The hole **6** through the length of the stopper **5** is filled by the inflation catheter **7.** The inflation catheter **7** in turn is connected at its distal end to a balloon or inflatable chamber **8.** A typical inflation catheter will have a diameter in the range 3 to 6 mm (often about 5 mm), although other diameters are possible as well. In preferred embodiments, the catheter will be (i) wide enough inside to lower resistance to fluid flow to the point that air can be used as the pressure medium, with a pressure source that need generate no more than 0.5 atmospheres in order to transmit pressure from the source to the balloon chamber, and (ii) narrow enough outside so that the presence of the inflation catheter in the various blood vessels does not significantly interfere with the flow of blood through the vessels. In this context, "narrow enough to avoid significant interference" means that the catheter occludes less than 50% of the vessel's lumen.

Each component may be constructed of any of a variety of well-known biocompatible materials, such as polytetrafluoroethylene (PTFE), expanded PTFE (ePTFE), polyurethane, polyethylene, polyethylene terephthalate, silicone, and titanium. The inflation catheter **7** and/or balloon **8** in particular may also beneficially comprise a moisture resistant material to help prevent water from blood passing through the balloon wall and building up in the chamber. For example, moisture resistance may be achieved by laminating a moisture resistant material onto or into the inflation catheter **7** and/or balloon **8**, or by applying moisture-resistant coating to the inner or outer surface of the balloon wall.

The stopper **5** may be useful in other ways besides preventing the build-up of tissue inside the graft **1.** The stopper **5** can act as a cushion surrounding the inflation catheter **7** so as to help maintain the inflation catheter's patency when the graft is tied down. Also, the increased surface area of the stopper **5** as compared to the inflation catheter **7** can ease the task of sealing the graft **1.**

Not shown in **FIG. 1** is the proximal end of the inflation catheter **7.** Because the balloon **8** needs to inflate and deflate in order to function as a ventricular assist device, the balloon pump must be in fluid communication with some sort of driver (e.g. an air compressor or pump) via the inflation catheter. If such a driver is to reside outside the body (as is typically done), a skin interface may be implanted. The skin interface, among other things, can help to decouple the internal parts of the pump assembly from the external parts. The inflation catheter can be attached to the interface, and the interface attached to the fluid driver. In this way, the driver, the inflation catheter **7** and the balloon **8** may form a closed air system; a closed system may include a well-defined and precisely-controlled volume of air, which facilitates leak detection. Air volume and movement of air may be precisely controlled using, for example, a bellows driven by one or more linear actuators. (In discussions of the skin interface and driver herein, the inflation catheter is alternatively referred to as an internal drive line.)

The arterial interface device of **FIG. 1** can be implanted in the body in a manner similar to the traditional intraaortic balloon pump described above. The graft **1** is attached to an artery **2** at an incision as described above. In addition to threading the balloon **8** and inflation catheter **7** through the graft **1**, the stopper **5** is positioned in the graft **1**, surrounding the inflation catheter **7.** The balloon **8** is positioned in the descending aorta and, if the stopper **5** is a separate piece from the inflation catheter **7**, the stopper **5** is positioned along the inflation catheter **7** so as to fill the distal end of the graft **1**, near where the graft **1** is attached to the artery **2.** The stopper **5** can be secured to the inflation catheter **7**, and graft **1** is secured to the stopper **5.**

To remove the balloon **8,** one simply detaches the stopper **5** from the graft **1.** Because the stopper **5** has prevented clots and other healing tissues from accumulating inside the graft **1**, the stopper **5** can be removed easily, leaving the graft **1** unblocked. The balloon pump can then be removed by pulling the inflation catheter **7** and balloon **8** through the graft 1 lumen. A new balloon pump can be advanced through the open graft **1** lumen along with a new stopper **5.** In this way, the balloon pump can be replaced without having to remove and replace the graft **1.** Because the vascular graft **1** is left intact and relatively undisturbed, no open surgery is necessary to replace a damaged or worn out part. Such a procedure is relatively non-invasive and can be carried out in a catheterization laboratory rather than an operating room.

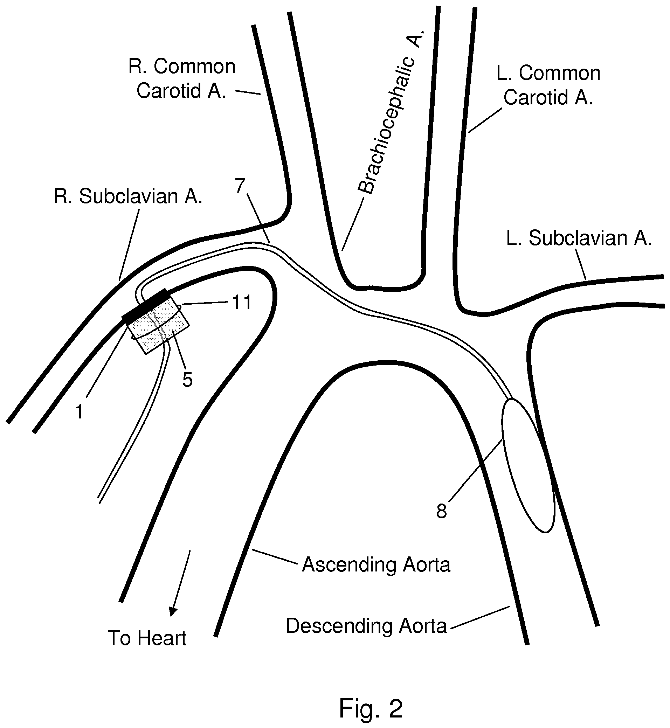

One of skill in the art will appreciate that many configurations of the stopper **5** are possible. The stopper **5** could be sized to completely fill the graft **1** surrounding the inflation catheter **7**, fitting snugly within the graft **1**, or the stopper **5** could be smaller than the interior of the graft **1** so that, for example, the graft **1** is cinched down onto the stopper **5** with a suture or tie. (Suture or tie **11** is shown in **FIG. 2**). The stopper **5** could be integrally formed with the inflation catheter **7.** The stopper **5** could have constant cross-sectional geometry, e.g., as a cylinder or prism, or the stopper **5** could be tapered or flared. The stopper **5** could be shaped to fit the interior of the particular vascular graft **1** being used. The stopper **5** could be made of two distinct pieces that form the entire stopper **5** when clamped together around the inflation catheter **7**, or the stopper **5** could be a single integral piece with a solid cross-section except for the hole **6** through which the inflation catheter **7** passes. The stopper **5** could be shaped with a circumferential notch **9** around its exterior to provide a convenient groove in which to run a tie or suture when securing the graft **1** to the stopper **5.** The stopper **5** could also include a circumferential ridge **10** around the interior surface that defines the hole 6, the ridge **10** acting as a seal between the stopper **5** and the inflation catheter **7.**

The hole **6** in the stopper **5** should be large enough to accommodate the inflation catheter **7**, but too narrow to pass the balloon **8.** Some outer dimension of the stopper **5** should be almost as large as, as large as, or larger than an outer dimension of the balloon **8** so that the balloon **8** can pass through the opening left after the stopper **5** has been removed without undue squeezing or compression. When in place, the stopper **5** should substantially fill the graft apart from the hole **6** for the inflation catheter. The hole **6** can account for various fractions of the smallest cross-sectional area of the stopper **5** including 75%, 60%, one half, one third, one quarter, or less.

**FIG. 2** shows (schematically) the vascular interface is position on the right subclavian artery. This position is advantageous because it allows easy surgical access and a relatively short distance to the descending aorta. **FIG. 2** also shows the graft secured to the stopper by a suture **11.** Other suitable positions for the interface include either common carotid artery, the brachiocephalic artery, the left subclavian artery, the descending aorta, and the abdominal aorta. Downstream branches of the aorta may also be used, such as the external iliac and femoral arteries.

In addition to the components shown in **FIGS. 1** and **2**, it may be beneficial for the device to include various sensors. Sensors located at or near the balloon chamber will typically be connected to an electrical wire that, like the inflation catheter, passes through the stopper **5** and graft **1.** The wire serves to pass the collected data out of the body, for instance to the fluid driver or an associated processor. Sensors that wirelessly transmit collected data are possible as well. Examples of sensors are electrical leads to measure the electrocardiogram, and sensors that detect pressure directly or indirectly. A wide variety of direct pressure sensors are known; the chamber itself can act as a pressure sensor when partially inflated. Indirect sensors include, for example, a microphone to monitor heart sounds. Data from these sensors can be used to monitor the cardiac cycle and, thereby, the counterpulsation cycle.

Sensors can also be used to determine the state of the air inside the system. Air pressure sensors can be used to detect whether the balloon pump is properly inflating, or if there is a leak in the system. A humidity sensor could be used to detect whether moisture has built up inside the balloon pump. The humidity sensor may be linked to a de-humidifier (such as an active dehumidifier or a vent system to exchange the pumping air with ambient air, described later) so that a certain level of humidity is not exceeded inside the balloon pump.

Sensors for arterial blood pressure may also be included, for example, at the pump or at the stopper. The sensors would communicate the detected arterial blood pressure by a signal the skin interface, either by wire or wirelessly. An arterial blood pressure monitor may similarly be located on the pump.

Although the drawings are directed to an intraaortic balloon pump, other indwelling arterial devices may be positioned using the disclosed arterial interface, such as indwelling arterial catheters ("A-lines"), dialysis lines, blood pumps such as axial flow pumps which add energy to flowing blood, and blood circulators such as those that remove blood from the aorta during systole and return it during diastole. While devices having distal ends larger than the catheters from which they extend may especially benefit, any device that may require replacement may benefit, as the stopper provides a convenient way to restore patency of the vascular graft for insertion of the replacement device.

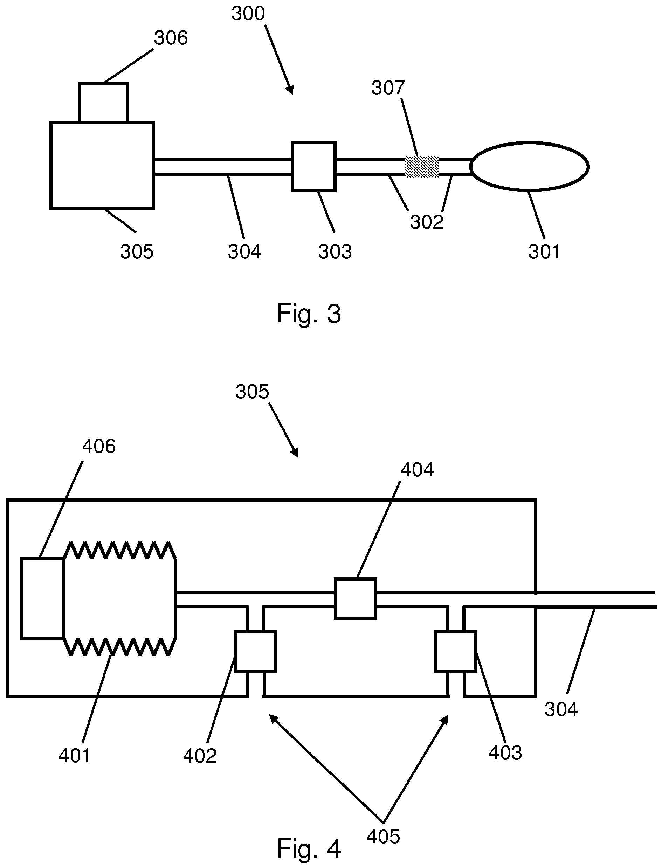

As described above, another improvement in ventricular assist devices is the improvement in portability achieved by eliminating the need for both a helium tank to supply pumping medium, and a compressor to dry the pumping medium. To this end, a ventricular assist device can include an intra-aortic balloon pump, an internal drive line, an arterial interface, a skin interface, an external drive line, an external driver, and a controller. One embodiment of such a ventricular assist device **300** is depicted schematically in FIG. 3.

The intra-aortic balloon pump **301** may be sized and shaped to dangle inside a patient's aorta. The wall of the balloon pump may include moisture resistant material, or may be entirely moisture resistant, to keep the air inside the balloon pump as dry as possible. One possible moisture resistant material for the balloon pump is polyurethane. The polyurethane polymers may be modified to include surface silicone end groups.

At its proximal end, the balloon pump **301** is connected to the distal end of the internal drive line 302. The skin interface **303** connects the proximal end of the internal drive line **302** to the distal end of the external drive line **304.** The proximal end of the external drive line **304** is connected to the driver **305.** The driver is connected to a controller **306.** An arterial interface **307** is sized and shaped to pass the internal drive line **302** through an arterial wall.

The balloon pump **301**, the internal drive line **302**, the skin interface **303**, the external drive line **304**, and the driver **305** can be charged with a pumping medium; a preferred pumping medium is air, but any fluid could be used. The balloon pump **301**, the internal drive line **302**, the skin interface **303**, the external drive line **304**, and the driver **305** can form a closed fluid system, or can be open, for example if the pumping medium is ambient air. In some embodiments, the balloon pump **301**, the internal drive line **302**, the skin interface **303**, the external drive line **304**, and the driver **305** can form a closed fluid system, or may operate in either a closed or open mode. The balloon pump **301** may have various sizes depending on the anatomy of the patient, but will typically have an inflated volume of about 40 to 60 cubic centimeters when inflated to 10 to 20 mmHg above the surrounding pressure.

FIG. 4 schematically depicts one embodiment of the driver **305.** The external driver includes a bellows **401** which may be rigid. The bellows **401** are in fluid communication with valves, **402, 403, 404** which are in turn connected to the external drive line **304.** The valves **402, 403, 404** can be controlled by the controller **306.** Bellows valve **402** is connected at one end to the bellows **401** and at the other end to ambient air **405.** Ambient air valve **403** is similarly connected on one end to the external drive line **304**, and at the other end to ambient air **405.** Pump valve **404** connects the bellows directly to the external drive line **304**, and eventually to the pump (not shown in FIG. 4). The amount of air expelled from the bellows **401** determines the pressure increase in the pump **301** during inflation. The bellows **401** are controlled to cause an increase in pressure in the pump **301** by a predetermined amount over the local aortic arterial blood pressure during diastole. The increase over local blood pressure could be in the range of 0 to 50 mmHg, for example 40 mmHg, but not exceeding a predetermined amount. The bellows **401** may have constant cross-sectional geometry along its length so that the volume is varied only by changing the length of the bellows **401.**

In a closed configuration, bellows valve **402** and ambient air valve **403** are left closed while pump valve **404** is left open. In this way, the balloon pump **301**, the internal drive line **302**, the skin interface **303**, the external drive line **304**, and the driver **305** form a closed fluid system. When bellows **401** contract, they pump air into the external drive line **304** and eventually into the balloon pump; when bellows **401** expand, air is drawn out of the balloon pump through the drive lines **302, 304** and back into the driver **305.** In this closed mode, no air is added to or vented from the device.

In an open configuration, ambient air **405** can be drawn into the system through one or both of valves **402** and **403.** The ambient air can be used to replace air already in the system, for example if the air in the system has become undesirably moist. Or ambient air **405** can be added to the air already in the system, for example if there is a leak in the system. Or ambient air can be used for the entire pumping cycle. Ambient air **405** can be drawn into the bellows **401** by opening bellows valve **402**, closing pump valve **404**, and then expanding the bellows **401** from a collapsed state. That same ambient air can then be forced into the external drive line **304** and thence into the pump **301** by closing bellows valve **402** and ambient air valve **403**, opening pump valve **404** and collapsing bellows **401.** Air can be vented or exhausted from the system by closing pump valve **404** and opening ambient air valve **403** when pump **301** is inflated to above the local blood pressure around pump **301.** In some embodiments, the system may have no pump valve and/or only one valve that can open to ambient air.

The bellows **401** can be actuated by a prime mover 406 controlled by the controller **306**. The prime mover can be any mover capable of contracting and expanding the bellows **401**. In one embodiment, the mover 406 is a screw turned by a motor; the motor rotates one way to advance the screw and compress the bellows **401**, and rotates the other way to cause the screw to retreat, expanding the bellows.

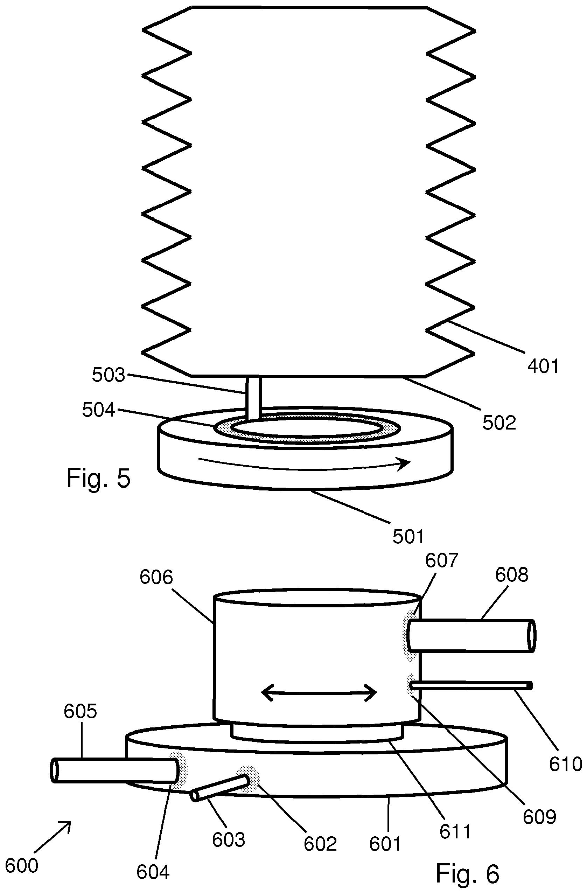

In another embodiment depicted in FIG. 5, a circular plate **501** is located near the moving surface **502** of the bellows **401**. One or more pegs **503** extend from the moving surface **502** and contact the plate **501** at a circular groove **504**. The depth of the groove **504** varies around its circumference. When the plate **501** rotates, the peg **503** rides up and down in the varying depth of the groove **504** causing the moving surface **502** of the bellows **401** to rise and fall. In this way, the shape of the groove **504**, e.g., its depth as a function of its circumference, its radius, etc., can be used to determine the frequency and amount of contraction and expansion of the bellows **401.** The plate **501** could include multiple grooves of varying geometry. In this way, a motor could be used to turn the plate **501** at a constant frequency, and the motion of the bellows **401** could be adjusted simply be moving the peg **503** from one groove **504** to another.

A controller **306** is programmed to operate the driver **305**, including expanding and contracting the bellows **401** by operating the prime mover **406**, and opening and closing the various valves **402**, **403**, **404** at the appropriate times, depending on the operating state of the driver **305.** In some modes, the valves **402**, **403**, **404** will be arranged so that as the bellows **401** expand, and ambient air **405** is drawn in to charge the device. In a closed mode, the controller **306** will close those valves **402, 403** that connect the interior of the system to the ambient air **405**, and operate the device using only the air with which the device is already charged. In general, the controller **306** will cause the driver to pump through multiple consecutive inflation-deflation cycles during the closed mode. The controller **306** may also receive signals from various sensors that may be part of the system, such as an arterial blood pressure sensor, an EKG, a microphone to monitor heart sounds, other types of monitors of cardiac activity, an air pressure sensor detecting the pressure of the air in the system, and/or a humidity sensor detecting moisture in the air in the system. Sensors could be deployed in various places, such as the pump **301**, either of the drive lines **302, 304**, the arterial interface **307**, the skin interface **303**, or at locations in the patient's body, not necessarily attached collocated with any part of the device.

When the driver **305** is operating in closed mode, the system will ideally maintain a constant volume of air. The device can include an air pressure sensor that senses the pressure inside the closed system, possibly as a function of time, and transmits a signal representative of that pressure to the controller **306.** The controller **306** can be programmed to receive a signal from the pressure sensor and compare the detected pressure to a predetermined normal operating range using a predetermined criterion or criteria. If the criteria are not met, the controller **306** can trigger an error condition. The controller **306** can be programmed to make the comparison using a variety of criteria. For example, the controller **306** could be programmed to trigger the error condition whenever the measured pressure is exceeds or falls below an upper or lower limit. Or the controller **306** could count the instances in which the measured pressure is outside a set range, and trigger the error condition only after a predetermined number of counts have accumulated. For example, the controller could calculate a rolling mean of the detected pressure, and the count could be increased each time the detected pressure is more than two standard deviations different than the mean pressure.

The controller **306** could also measure the amount of time necessary for the balloon pump **301** to inflate once based on the sensed air pressure as a function of time. If the controller detects that the time needed to inflate the balloon pump **301** is shorter or longer than a target time, the controller **306** can lower or raise respectively the amount of power provided to the driver **305** so as to adjust the inflation time to meet the target time. The controller **306** may be programmed to calculate an adjustment to the driver power based on the deviation of the actual inflation time from the target inflation time. The controller **306** may be programmed to include a minimum and maximum power to be input to the driver **305**, regardless how the measured inflation time compares to the target inflation time.

The controller **306** can also be programmed to detect air leaks in the system. The constancy of the amount of air in the system can be checked by determining the pressure at a particular time in the pumping cycle, and comparing to the pressure at the same point in previous pumping cycles. If the pressure is the same from cycle to cycle, then the amount of air in the system is not changing. If the pressure has dropped in later cycles, then air must be leaving the system. In this way, an air pressure sensor can be used to detect a leak in the system.

A sensor, such as a humidity sensor, could be connected so as to transmit a signal to the controller **306.** The controller **306** could be programmed to receive the signal and determine, based on the signal, whether to operate the driver in closed mode or open mode. In particular, if the sensor is a humidity sensor, the controller **306** could detect that the air in the system has become moist, e.g., during operation in closed mode. The controller **306** could be programmed to switch the driver **305** to open mode in order to exchange moist air in the system with relatively dry ambient air. The controller **306** could be programmed to then switch the driver **305** back to closed mode once the humidity inside the system has achieved an acceptable level.

An EKG sensor could be deployed in the patient so as to detect an EKG signal, which could then be transmitted to the controller **306.** The device could also include a pressure sensor that detects or infers a ventricular pressure and is coupled to the controller **306** so as to transmit a signal to the controller **306.** The controller **306** could be programmed to use the EKG signal to detect a QRS complex and the pressure signal to detect a dicrotic notch. Various algorithms and methods for QRS detection and dicrotic notch detection are discussed in Hamilton and Tompkins, Quantitative Investigation of QRS Detection, Rules Using the MIT/BIH Arrhythmia Database, IEEE Transactions on Biomedical Engineering, Vol. BME-33, No. 12, December 1986, Kantrowitz, Introduction of Left Ventricular Assistance, ASAIO Journal, Vol. 10, No. I, January-March 1987, and Pan and Tompkins, A Real-Time QRS Detection Algorithm, IEEE Transactions on Biomedical Engineering, Vol. BME-32, No. 3, March 1985. The controller
306 could be programmed to trigger inflation of the pump following the dicrotic notch and deflation of the pump following the QRS complex. The controller **306** could be programmed: first to enter a conservative mode in which the controller **306** detects the QRS complex in order to trigger deflation, but triggers inflation based on other information, for example a guess as to the timing of the dicrotic notch relative to the QRS complex; and second to enter a normal mode in which the controller **306** triggers inflation based on actual detection of the dicrotic notch.

The volume of air needed to achieve the desired pressure inside the balloon pump during diastole can be determined with a searching algorithm. The algorithm can cause the bellows to compress a variety of different amounts during various diastoles, e.g., 75% during a first diastole, 80% during the next diastole, 85% during the next, etc. The pressure in the drive line and/or in the balloon pump can be recorded throughout each of these cycles and analyzed to determine which degree of bellows compression corresponds to a desired pressure increase.

In various embodiments, a ventricular assist device can include an external drive unit with any one of, or any combination of, a variety of features. The drive unit can be a small box designed to be worn exterrnally by a patient. The drive unit can include a rechargeable battery, a transformer, a custom circuit board, custom software, and one or more valve manifolds.

A skin interface for a ventricular assist device may include a first portion, at least partially internal to, and a second portion external to the patient, that are fixed to one another, but rotatable with respect to one another. One possible embodiment of such a skin interface is shown schematically in FIG. 6. It is possible to implant such a skin interface in a variety of different locations on the patient, for example abdominally or thoracically.

The skin interface **600** has an internal portion **601** that may be implanted in the patient so that at least a part is subcutaneous. The internal portion **601** includes at least one receptacle **602** for receiving one or more internal electrical lines **603** and a receptacle **604** for an internal air line **605**. If there are multiple internal electrical lines **603**, they may be received in separate receptacles or a single receptacle. The internal electrical line or lines **603** may be connected to one or more sensors. The internal electrical lines **603** can be configured so as to transmit a signal from one or more sensors, such as an arterial blood pressure sensor, an air pressure sensor, or an EKG sensor, to a processor in the skin interface **600**. If the skin interface **600** includes a processor, the processor may be programmed to receive signals through the internal electrical lilies **603** from the sensors and output the signals. The processor may digitize the signals and produce a digital output indicative of the input received from the sensor or sensors. The skin interface **600** may also include a memory in which patient-specific parameters are stored. The internal air line **605** (or internal drive line) is connected to a balloon pump (not shown) dangling in the patient's aorta.

The skin interface **600** also has an external portion **606.** The external portion **606** also includes a receptacle **607** for an external air line **608** and one or more receptacles **609** for one or more external electrical lines **610.** The external air line **608** (or external drive line) is connected to an external driver (not shown). The external electrical line **610** may be connected to a processor or a memory in which patient-specific data are stored, both contained in the skin interface **600.** The external electrical lines **610** can receive the output from the processor. The external electrical lines **610** may also be connected with the memory so as to allow input to and output from the memory through the external electrical lines **610.** The memory could be used to store data accumulated during normal operation of the ventricular assist device, or information obtained during a doctor's visit. The information may be accessed either by a doctor, for example to investigate the past performance of the ventricular assist device or to obtain data on the patient's health as detected by sensors. Or the information may be accessed by a processor in an external driver, for example to set parameters for operation of the ventricular assist device.

The internal and external portions **601**, **606** are fixed to one another so that they remain attached to each other but are rotatable with respect to one another. In this way, the internal portion **601** can remain stationary with respect to the patient while the external portion **606** can be rotated to accommodate any convenient orientation of the external air line **608** and external electrical line or lines **610.** Such rotational decoupling can help reduce or prevent tugging or other stress on the patient's skin or other organs. The internal and external portions **601**, **606** are coupled so as to create an air-tight conduit between the internal and external air line receptacles **604**, **607.** In this way, the internal and external air lines **605**, **608** can be part of a closed fluid system. In one embodiment, an air-tight seal is formed by fixing the internal and external portions **601**, **606** to one another using magnets. Gaskets and other sealing systems may be used. The internal and external portions **601, 606** also couple the internal and external electrical line receptacles **602, 609** so as to allow transmission of electrical signals and power through the skin interface **600.** Such transmission may be wireless, for example by infrared signals. The skin interface **600** may include a biocompatible surface and/or a finish that promotes biological ingrowth. The internal and external portions **601**, **606** may be separated by a medial portion **611.**

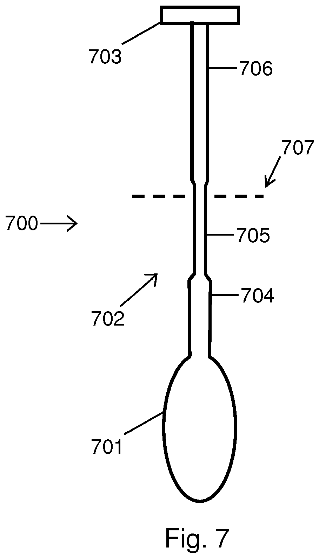

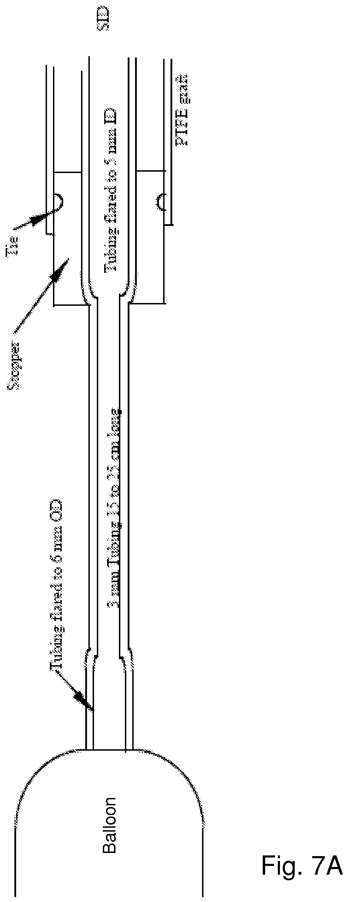

An intra-aortic balloon pump assembly can include a balloon pump and a drive line with regions of varying cross-sectional area and/or diameter. The size and/or cross-sectional shape of the line is varied to avoid occluding an artery in which part of the line is deployed, while also allowing air to flow effectively through the line. The larger the internal cross-section of the drive line, the easier it is to force air through the drive line. So it is preferable to design a drive line with a large inner diameter or cross-sectional area for as much of its length as possible. On the other hand, where the drive line is deployed within an artery, the line should not be so large as to occlude the artery to the point that the artery cannot provide minimal essential blood flow to downstream tissues. One embodiment of such a balloon pump assembly is shown schematically in FIG. 7.

The exemplary assembly **700** shown in FIG. 7 includes an intra-aortic balloon pump **701** sized and shaped to dangle inside a patient's aorta, and a drive line **702** with three regions. The drive line **702** forms an air tight connection with the balloon pump **701** at the line's distal end, and connects to a skin interface **703** at the line's proximal end. The drive line **702** has a pump region **704** at its distal end, adjacent to the balloon pump **701.** In the middle, the drive line **702** has an arterial region **705.** At its proximal end the drive line **702** has an extravascular region **706.** The balloon pump **701** may be made at least in part from a moisture-resistant material, such as polyurethane modified with hydrophobic end groups. The hydrophobic end groups could be silicone groups.

The assembly shown in FIG. 7 is designed to be deployed in the body of a patient, extending from a skin interface, through the wall of an artery such as the subclavian artery, into the aorta, where the balloon pump **701** dangles. The drive line **702** can enter the artery through an arterial interface such as those described above. When deployed in the body, the extravascular region **706** extends from the skin interface to near the artery. The arterial region **705** is located generally in the artery and may extend beyond the artery. The pump region **704** is deployed generally in the aorta. The artery wall **707** is schematically represented by the dashed line.

The extravascular region **706** may be designed to have relatively large cross-section to improve air flow. Because the extravascular region **706** of the drive line **702** is not intended to be deployed inside an artery, there is less motivation to minimize the size of the line. The extravascular region **706** may be, for example 4 to 8 mm inner diameter, and in particular 6 mm inner diameter. The pump region **704** may be similarly designed to have a relatively large cross-section, since it is intended to be deployed in the aorta, which is itself large and is unlikely to be occluded by the drive line **702**; for example the pump region may have an outer diameter of 6 mm where it meets the pump, so that the pump region **704** is sized and shaped to form an air tight connection with the pump **701.** But the arterial region **705** is intended to be deployed in an artery such as the subclavian, and as such it should have an outer diameter sufficiently small to avoid occluding the artery. An arterial region with a cross-sectional area less than 50% of the internal cross-sectional area of the artery is preferred. For the subclavian artery for example, the arterial region **705** could have an outer diameter of about 5 mm. As noted above, the drive line could placed be in any of a variety of arteries, and the geometry of the arterial region would have to be adapted to the particular artery in question. The pump region **704** may be less than 6 cm in length, in particular about 2 to 4 cm, to reach from the orifice of the subclavian artery to where the balloon is intended to rest in the descending aorta. The arterial region **705** could be less than 20 cm in length, in particular about 8 to 15 cm. The extravascular region **706** could be any length necessary to reach from the artery to the skin interface **703**, a typical length being about 25 cm. All the dimensions cited above for the various parts of the drive line **702** will depend on the particular anatomy of the patient, the particular artery through which the assembly is deployed, the location of the skin interface, and similar geometrical considerations. The diameters of the pump region **704** and extravascular region **706** could be, but need not be, different from each other. The diameters of the pump region **704** and the extravascular region **706** may be larger than the diameter of the arterial region **705.**

**FIG. 7A** schematically shows another embodiment of a balloon assembly with a drive line having regions of differing diameter; exemplary dimensions are marked.

In some embodiments, an entire internal drive line and balloon pump may be a single integrally formed piece, and may even include the arterial interface described above. In that case, if and when the balloon pump is replaced, the entire drive line could be replaced down to the skin interface as well. Or the internal drive line could be cut somewhere between the skin interface and the balloon pump, and a new balloon pump and portion of the internal drive line attached to an unreplaced portion of the internal drive line. The old and new portions of the drive line could be attached in any of a variety of ways, for example, adhesives or hose barbs. In other embodiments, the internal drive line may not be integral so that a physician may disconnect parts of the drive line to replace them, leaving other parts of the drive line in place.

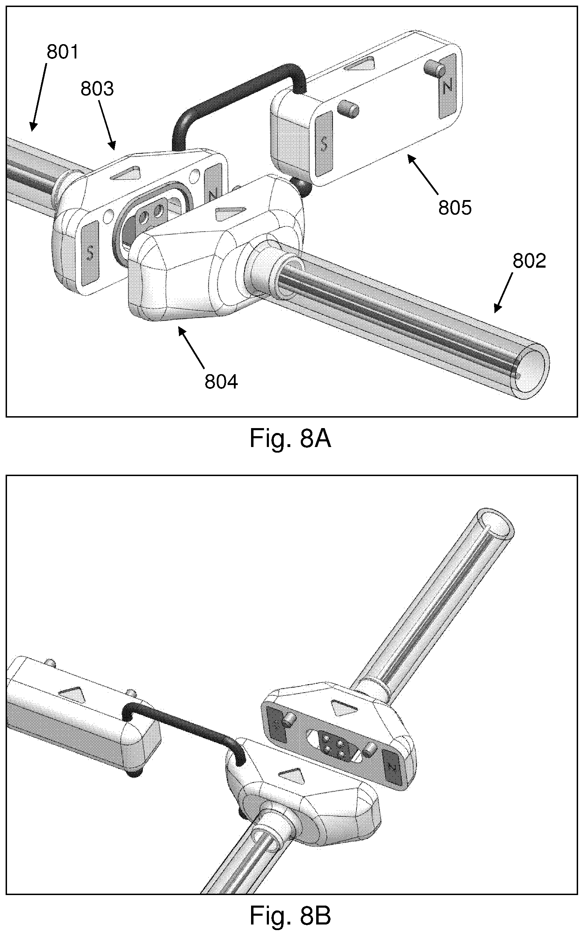

A ventricular assist device can include an external drive line with a detachable patient connector. One possible embodiment of a patient connector is shown in two views in FIGS. 8A & 8B; the figures show the patient connector disconnected for clarity. An external drive line integrated with electrical wires is shown in two parts, a patient-side external drive line **801**, and a driver-side external drive line **802.** At one end, the patient-side external drive line **801** connects to the patient side of the patient connector **803.** At the other end, the patient-side external drive line **801** is connected to the external portion of a skin interface (not shown) implanted in the patient. At one end, the driver-side external drive line **802** connects to the driver side of the patient connector **804**. At the other end, the driver-side external drive line **802** is connected to an external driver (not shown). Mating magnets marked "N" and "S" affix the two sides **803**, **804** of the patient connector to each other to form an air tight seal. The two sides **803**, **804** of the patient connector also form electrical connections for the wires so that signals and power may be transmitted from one side of the connector to the other. By using magnets, the sides of the patient connector are easily attachable and detachable, allowing a patient to disconnect or reconnect the implanted device from an external driver. After disconnecting the two sides **803**, **804** of the patient connector, the patient may seal off the patient-side external drive line with a cap, 805, which also includes mating magnets.

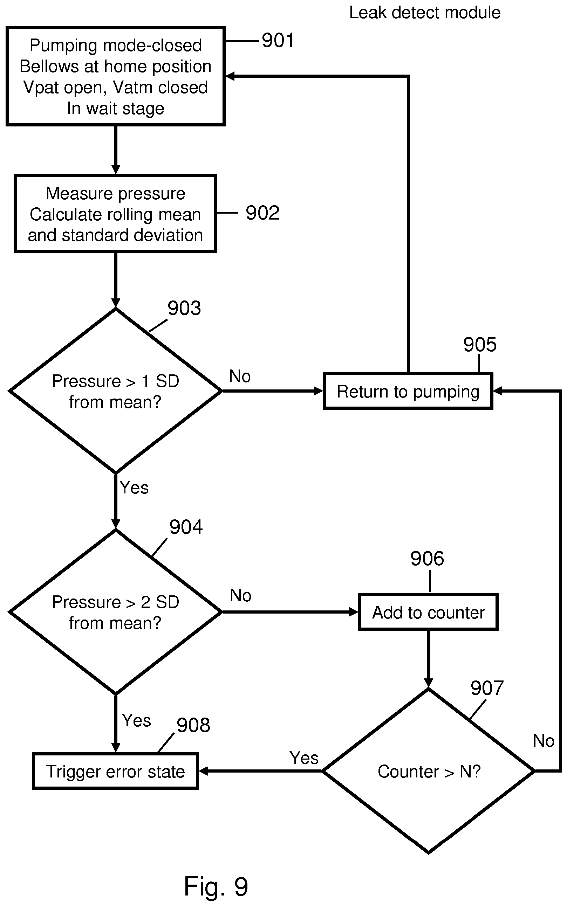

As described above, software can be used to control a variety of functions of an external driver in an intraaortic balloon pump system. FIGS. 9 - 14 describe several such functions. FIGS. 9 - 14 represent only examples of how software might be used to control such a system, and are not intended to be limiting.

FIG. 9 describes a leak detection function. The system starts by pumping in closed mode **901**, and calculates a rolling mean and standard deviation of the measured air pressure inside the balloon pump and/or drive lines at a particular point in each pumping cycle **902.** The measured pressure at the chosen point in the pumping cycle is then compared to the mean pressure **903**, **904.** If the measured pressure is less than one standard deviation away from the rolling mean pressure **903**, then the system simply returns to pumping without any further action **905.** If the measured pressure is more than one standard deviation away from the rolling mean pressure **903**, then the software will ask whether the measured pressure is more than two standard deviations from the rolling mean pressure **904.** If not, the system increments an error counter **906.** If the error counter has yet to reach a threshold level N **907**, then the system returns to normal pumping **905.** If the error counter has passed the threshold, then the system triggers an error state **908.** Likewise, if the measured pressure is more than two standard deviations from the rolling mean pressure **904**, then the system triggers an error state **908.**

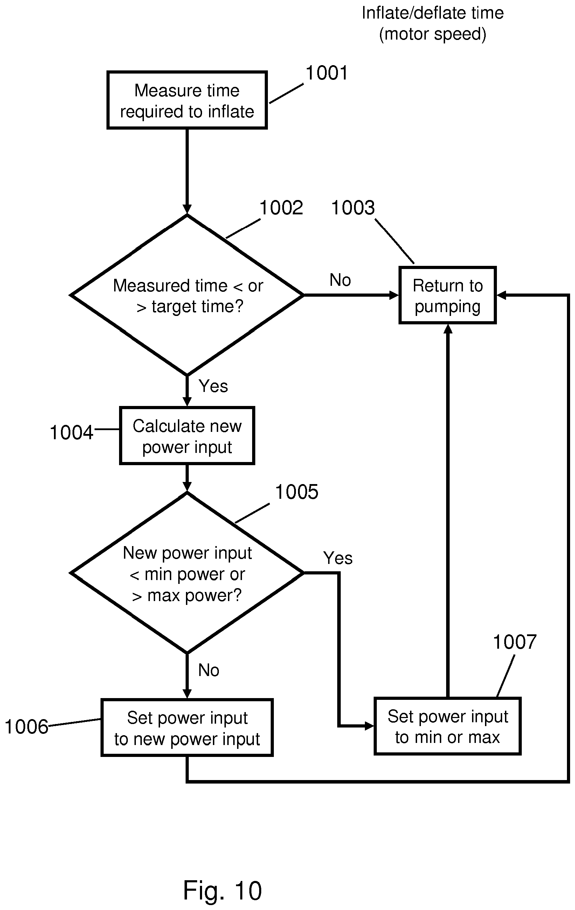

FIG. 10 describes a function to adjust the time taken to inflate the balloon pump. The system starts by measuring the time taken to inflate the pump **1001.** The system then compares the time to a target time **1002.** If the measured time is neither significantly less nor greater than a target time, i.e. the measured time matches a target time, then the system continues to operate with no change in power input to the driver **1003.** If the measured time is significantly different than the target time, then the system calculates the power necessary to cause the inflation time to match the target time **1004.** If the pump is inflating too slowly, i.e., if the inflation time is significantly greater than the target time, the power supplied to the driver should be increased; if the pump is inflating too quickly, i.e., if the inflation time is significantly less than the target time, the power supplied to the driver should be reduced. After calculating a new power level, the system compares the new power level to the minimum and maximum allowable power levels for the driver **1005.** If the new calculated power level is between the minimum and maximum allowable power levels for the driver, the system sets the input power level to the calculated level **1006** and returns to pumping **1003.** If the new calculated power level is less than the minimum or greater than the maximum allowable power levels, the system sets the power level to the minimum or maximum respectively **1007** and returns to pumping **1003.**

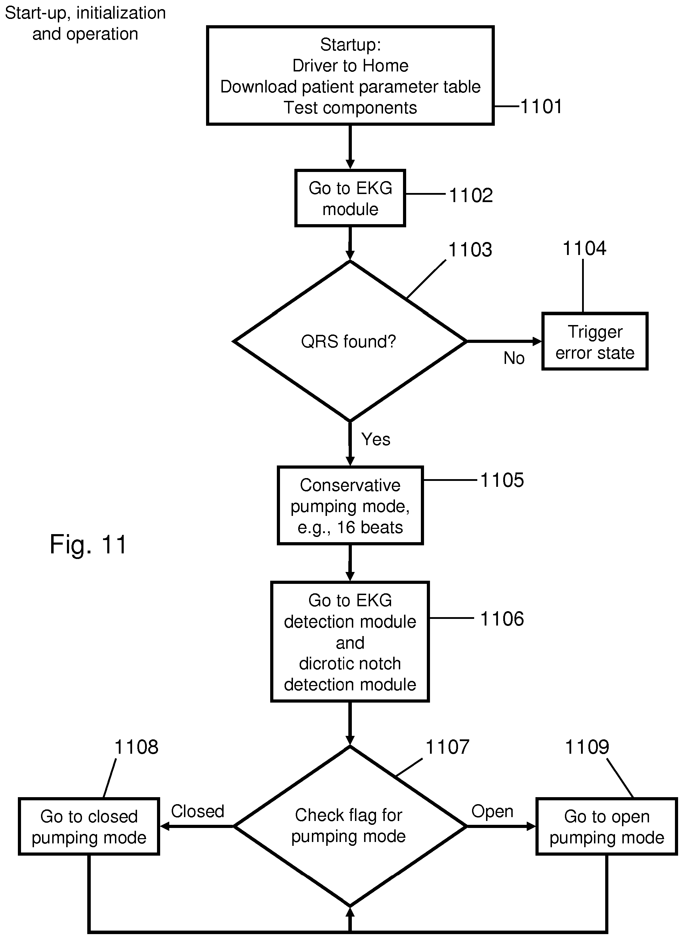

FIG. 11 describes a method of initializing and starting up the system. On powering up the system, the driver and its actuator are sent to a home position, a patient parameter table is downloaded if available, and tests are run on system components that could include a watchdog timer, memory including non-volatile memory, non-volatile static RAM, ROM, a temperature sensor, pressure transducers, and a battery **1101.** At this time, the system may also pressurize to check for leaks. Then an EKG detection module is initiated **1102,** which is described in more detail below. If the QRS complex is not successfully detected **1103** by the EKG detection module, then an error state is triggered **1104.** If the QRS complex is successfully detected, then a conservative pumping mode is activated for a limited time, say 16 beats **1105.** In conservative mode, inflation and deflation are triggered based on a mean "RR interval" (the time between subsequent R-wave peaks) and an expected delay between the R-wave peak and the dicrotic notch; the dicrotic notch is not directly detected. After starting in conservative mode, the system then moves to detect both the QRS complex and the dicrotic notch **1106** to trigger deflation and inflation respectively. Once the QRS complex and dicrotic notch have been successfully detected, the system checks a flag **1107** to see which pumping mode, open or closed, is desired. The system then enters the appropriate mode **1108, 1109** and cycles back to check the flag again **1107** in the next pumping cycle. Open and closed modes **1108, 1109** are described in more detail below.

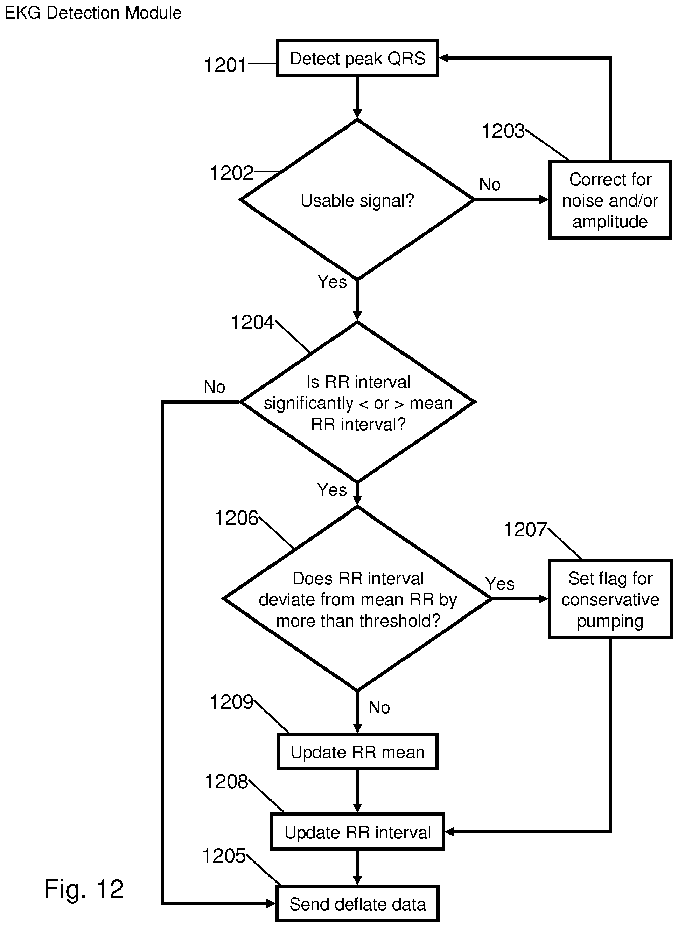

FIG. 12 describes a method of using an EKG signal to time the QRS complex. First the system detects the peak signal of the QRS complex **1201**, the R-wave. One example R-wave detection scheme could work as follows. EKG signal peaks will be classified as potential R-wave peaks if (1) the amplitude of the signal exceeds 50% of average signal value and (2) if the slope of the signal exceeds 25% of the average maximum point to point value within a segment. Potential R-wave peaks could be rejected if they occur within 250 milliseconds of a previous R-wave. To reject erroneous T-wave triggers, the slope threshold above could be increased to 50% during the 400 milliseconds following a previous R-wave. Any potential R-waves not rejected in this way could be considered detections of the R-wave. If the signal is of poor quality **1202** the system can correct for noise **1203** and restart the QRS detection **1201.** If the signal is usable and an R wave is detected, the system will calculate the current RR interval and compare it to the mean RR interval **1204.** If the current RR interval is not significantly different than the mean, the system sends a signal to deflate the balloon pump **1205.** If the current RR interval is significantly less than or greater than the mean, then the system checks whether the current RR interval deviates from the mean by more than a predetermined threshold amount **1206.** If so, the system goes into conservative pumping mode **1207** subtracting a programmed time interval from the calculated time to deflate. The system updates the previously calculated mean RR interval **1208** and sends the deflation signal **1205.** If the current RR interval deviates from the mean by less than a predetermined threshold amount **1206**, then the current RR interval is used to update the running mean RR interval **1209**, the stored, latest RR interval is updated **1208** and the deflation signal is sent **1205.** The system may also use the measured RR interval and its change over time to detect arrhythmia. The system may also detect pulses from a pacemaker and blank those false signals out from the QRS detection scheme.

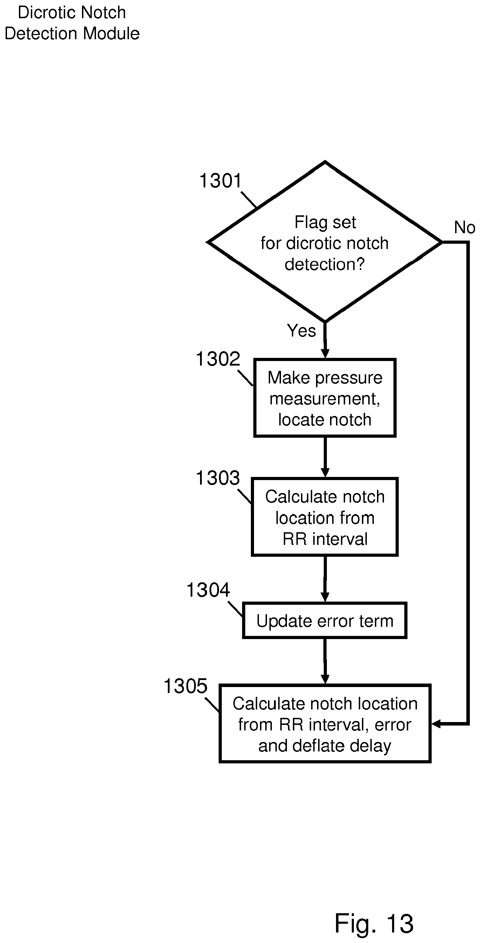

FIG. 13 describes a method of detecting a dicrotic notch. First, the system will check to see whether a flag is set to indicate whether dicrotic notch detection is indicated **1301.** If so, the system makes a pressure measurement to time the dicrotic notch **1302.** The dicrotic notch could be detected directly, for instance by a pressure transducer in the aorta, or indirectly, for example by measuring the effect of the ambient arterial blood pressure on the air in the balloon pump. Regardless of the sensor arrangement, multiple criteria are used to determine the dicrotic notch timing, with a statistical weight for each criterion. The primary criterion will be a positive slope in the pressure as a function of time, or if no positive slope is detected, the least negative slope. Notch detection can be improved by only looking for the notch in an appropriate time window. One estimate of the length of time from R-wave peak to dicrotic notch can be calculated as the QS2 interval (based on a second order polynomial); a useful window for dicrotic notch searching would be centered at the end of the calculated QS2, and extend plus or minus some fraction of QS2, for example 25%. When the dicrotic notch is located **1302**, the distance of the notch from the R-wave peak is also calculated **1303.** The error, i.e. the difference between the QS2-predicted-timing of the dicrotic notch and the actual timing of the dicrotic notch, is also calculated **1304**. Based on the distance of the dicrotic notch from the R-wave and the error term, an updated QS2 is then calculated **1305** to be used to estimate the location of the dicrotic notch in the subsequent beat. Constantly updating the QS2 interval and the error term can allow the system to better follow rapidly changing heart rates. Also, if dicrotic notch detection failed entirely, the calculated QS2 plus a previous error term would provide a backup estimate of the timing of the dicrotic notch.

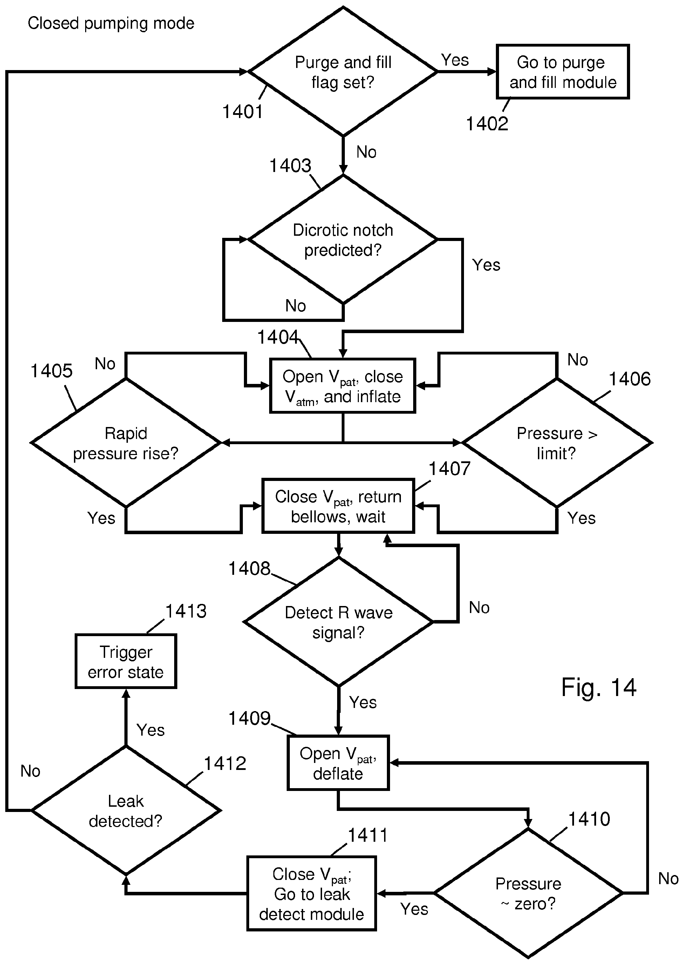

FIG. 14 describes how the system can run in a closed pumping mode. First the system checks whether a purge-and-fill flag is set **1401.** If so, then the system enters a purge-and-fill mode **1402** described in detail below. If not, the system continues into normal closed pumping mode. If the system has not predicted dicrotic notch **1403**, then the system continues to wait; when a dicrotic notch has been predicted, the system proceeds to open Vₚₐₜ (one or more patient-facing valves, e.g., the pump valve **404** of FIG. 4), close Vₐₜₘ (one or more valves open to ambient air, e.g., bellows valve **402** and ambient air valve **403** of FIG. 4), and begins inflating the balloon **1404**. The system then checks two conditions, rapid pressure rise **1405**, and a pressure limit **1406**. If the pressure rise is not overly rapid **1405**, and if the pressure does not reach a limit value **1406**, then the system continues inflation **1404**. If either condition **1405**, **1406** is met then the system closes Vₚₐₜ and returns the bellows to a neutral position **1407**. With the balloon pump inflated, the system then checks whether an R-wave is detected **1408**. If no R-wave is detected the system continues to wait **1407**. If an R-wave is detected, the system opens Vₚₐₜ and begins deflating the balloon pump **1409**. The system then checks whether the air pressure in the system has reached roughly zero, i.e. whether the air pressure in the balloon pump has equilibrated with the surrounding blood pressure **1410**. If not, the system continues to deflate the balloon pump **1409**; if so, the system closes Vₚₐₜ and proceeds to the leak detection module described above **1411**. If a leak is detected **1412**, then the system triggers an error state **1413**. If no leak is detected, the system returns to the beginning of the cycle to check whether the purge and fill flag has been set **1401**. This process is further explained in Table 1:

**Table 1**

| Stage | Event | Start | End | Vₚₐₜ | Vₐₜₘ | Bellows and actuator |
|---|---|---|---|---|---|---|
| 1 | Deflation | R-wave plus deflation delay | Bellows in home position (pressure reaches roughly zero) | Open | Closed | Return to home, deflate |
| 2 | Wait | Bellows reach home position | Dicrotic notch plus inflation delay | Open | Closed | Wait |
| 3 | Leak Detection | As soon as stage 2 begins | When leak detection module finishes | Closed | Closed | Wait |
| 4 | Inflation | Dicrotic notch plus inflation delay | Rapid rise in pressure or pressure past limit | Open | Closed | Actuate, compress and inflate |
| 5 | Wait | Rapid rise in pressure or pressure past limit | R-wave plus deflation delay | Closed | Closed | Wait |

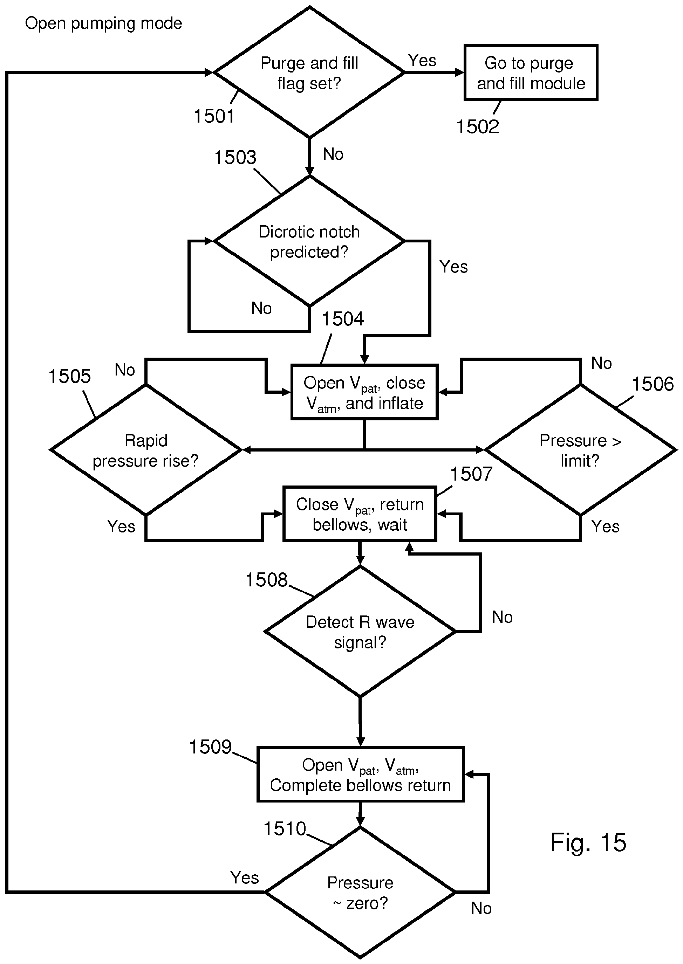

FIG. 15 describes how the system can run in a open pumping mode. First the system checks whether a purge-and-fill flag is set **1501.** If so, then the system enters a purge-and-fill mode **1502** described in detail below. If not, the system continues into normal open pumping mode. If the system has not predicted dicrotic notch **1503**, then the system continues to wait; when a dicrotic notch has been predicted, the system proceeds to open Vₚₐₜ, close Vₐₜₘ, and begins inflating the balloon **1504**. The system then checks two conditions, rapid pressure rise **1505**, and a pressure limit **1506**. If the pressure rise is not overly rapid **1505**, and if the pressure does not reach a limit value **1506**, then the system continues inflation **1504**. If either condition **1505, 1506** is met then the system closes Vₚₐₜ and returns the bellows to a neutral position **1507**. With the balloon pump inflated, the system then checks whether an R-wave is detected **1508.** If no R-wave is detected the system continues to wait **1507.** If an R-wave is detected, the system opens Vₚₐₜ and Vₐₜₘ, and returns the bellows to the home position **1509.** By opening Vₐₜₘ, the balloon is allowed to deflate, exhausting the air with which it was inflated **1504.** At the same time, because the compressed bellows are decompressed with Vₐₜₘ open, the bellows draw in fresh ambient air. The system then checks whether the air pressure in the system has reached roughly zero, i.e. whether the air pressure in the balloon pump has equilibrated with the surrounding blood pressure **1510.** If not, the system continues to allow the balloon pump to deflate while the bellows draw in fresh air **1509**; if so, the system returns to the beginning of the cycle to check whether the purge and fill flag has been set **1501.** This process is further explained in Table 2.

**Table 2**

| Stage | Event | Start | End | Vₚₐₜ | Vₐₜₘ | Bellows and actuator |
|---|---|---|---|---|---|---|
| 1 | Deflation | R-wave plus deflation delay | Bellows in home position (pressure reaches roughly zero) | Open | Open | Return to home, deflate |
| 2 | Wait | Bellows reach home position | Dicrotic notch plus inflation delay | Open | Open | Wait |
| 3 | Inflation | Dicrotic notch plus inflation delay | Rapid rise in pressure or pressure past limit | Open/Closed* | Closed | Actuate, compress and inflate |
| 4 | Wait | Rapid rise in pressure or pressure past limit | R-wave plus deflation delay | Open/Closed* | Closed | Wait |

| | | | | | | |
|---|---|---|---|---|---|---|
| * If there are multiple Vₚₐₜ valves, they may be closed during the first wait and then opened in pairs with a predetermined delay, e.g., 20 milliseconds, between openings. This may be discontinued at higher heart rates or when the actuator motor is operating at maximum speed | | | | | | |

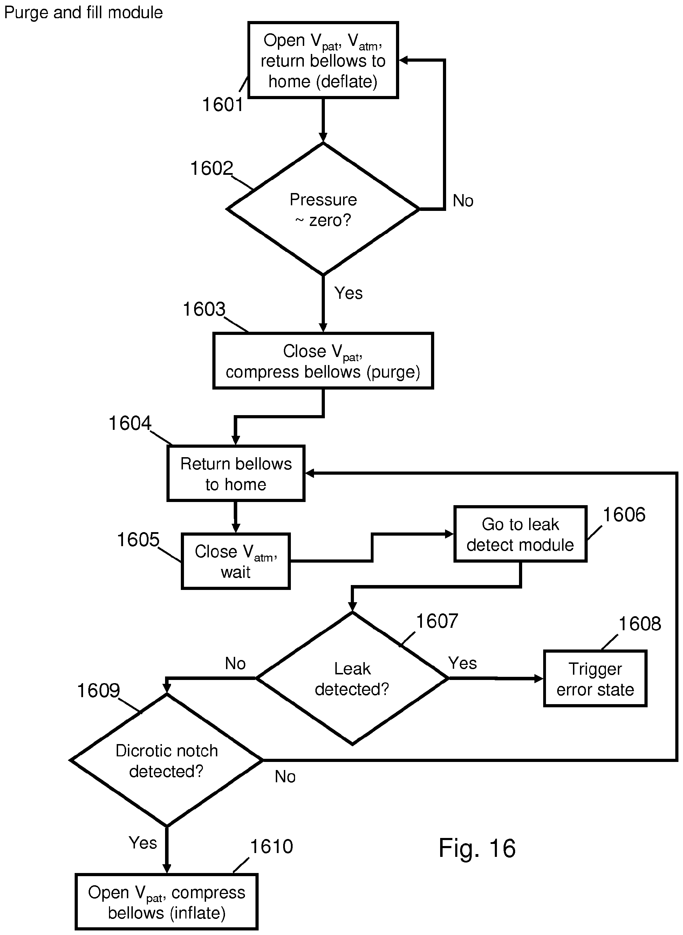

FIG. 16 describes a method of purging air from the system and replacing it with ambient air. First the system opens both Vₚₐₜ and Vₐₜₘ, and returns the bellows to the home position in order to fully deflate the balloon pump **1601.** If the pressure in the system has not reached roughly zero **1602**, then the system continues to expand the bellows and leave the valves open. When the pressure has reached roughly zero, the system closes Vₚₐₜ and compresses the bellows with Vₐₜₘ still open, so as to purge the air from the bellows **1603.** No air is sent to the balloon pump at this time because Vₚₐₜ is closed. The bellows are then returned to home position, again drawing fresh air into the bellows **1604.** Once the bellows have been filled with ambient air, the system closes Vₐₜₘ **1605** and enters the leak detection module **1606.** If a leak is detected **1607**, the system triggers an error state **1608**; otherwise the system checks whether a dicrotic notch has been detected or predicted **1609.** If not, the system again returns the bellows to home **1604** and waits for a dicrotic notch detection. If a dicrotic notch is detected **1609**, then the system opens Vₚₐₜ and compresses the bellows with Vₐₜₘ still closed **1610**, driving the fresh ambient air from the bellows into the balloon pump. From this state, the system could enter normal closed pumping mode. Table 3 further describes how a purge/fill module could be implemented.

**Table 3**

| Stage | Event | Start | End | Vₚₐₜ | Vₐₜₘ | Bellows and actuator |
|---|---|---|---|---|---|---|
| 1 | Deflation | R-wave plus deflation delay | Bellows in home position | Open | Closed | Return to home, deflate |
| 2 | Purge | Bellows reach home position | Pressure reaches roughly zero | Closed | Open | Compressing |
| 3 | Fill | Dicrotic notch plus inflation delay | Bellows in home position | Closed | Open | Return to home |
| 4 | Wait | Bellows reach home position | Dicrotic notch plus inflation delay | Open | Closed | Wait |
| 5 | Leak Detection | As soon as stage 4 begins | When leak detection module finishes | Closed | Closed | Wait |
| 6 | Inflation | Dicrotic notch plus inflation delay | Rapid rise in pressure or pressure past limit | Open | Closed | Actuate, compress and inflate |

## Claims

1. A ventricular assist device (300), comprising:
an intra-aortic balloon pump (301) sized and shaped to dangle inside a patient's aorta, the pump (301) having a wall made at least in part from a moisture-resistant material;
a skin interface (303);
an external driver (305) comprising a rigid bellows (401), a prime mover (406) for actuating the bellows (401), and valves arranged to permit multiple pumping modes;
an internal drive line (302) connecting the balloon pump (301) to the skin interface (303);
an arterial interface (307), sized and shaped to pass the internal drive line (302) through an arterial wall;
an external drive line (304) connecting the skin interface (303) to the driver (305); and a controller (306);
wherein
the balloon pump (301), the internal drive line (302), the skin interface (303), the external drive line (304), and the driver (305) form a fluid system for containing a pumping medium; and
the controller (306) is programmed to:
operate the valves (402, 403, 404) and prime mover (406) such that the pumping medium is not pressurized more than a predetermined amount above a sensed blood pressure;
cause the valves (402, 403, 404) to adopt a closed pumping mode in which no pumping medium is added to or vented from the device (300);
cause the driver (305) to pump through multiple consecutive inflation-deflation cycles during the closed mode;
**characterized in that**:
the pumping medium present in the fluid system comprises ambient air (405), and the controller (306) is programmed to cause the valves (402, 403, 404) to adopt an open mode in which the pumping medium is vented from the fluid system and replaced with ambient air (405).

2. The device (300) of claim 1 wherein, in the closed pumping mode, the balloon pump (301), the internal drive line (302), the skin interface (303), the external drive line (304), and the driver (305) form a closed fluid system charged with a column of pumping air that acts as the pumping medium;
the device (300) further comprising a pressure sensor that detects the air pressure inside the closed fluid system, the pressure sensor being so connected to the controller (306) as to allow transmission of a signal representative of the pressure to the controller (306); and wherein the controller (306) is programmed to receive the signal from the pressure sensor;
compare the pressure of the air column to a predetermined normal operating range by a predetermined set of operating criteria; and
trigger an error condition if the pressure is outside the normal operating range.

3. The device (300) of claim 2, wherein the controller (306) is further programmed to:
calculate a rolling mean and a rolling standard deviation of the air pressure of the pumping air at a particular point in the inflation-deflation cycles based on signals from the air pressure sensor at the particular point in multiple consecutive inflation-deflation cycles; and
calculate a difference between (a) an air pressure measured at the particular point in the current inflation-deflation cycle and (b) the rolling mean of the air pressure;
wherein the error state is triggered if the difference is greater than twice the rolling standard deviation of the air pressure.

4. The device (300) of either of claims 2 or 3, wherein the controller (306) is further programmed to:
calculate a rolling mean and a rolling standard deviation of the air pressure of the pumping air at a particular point in the inflation-deflation cycles based on signals from the air pressure sensor at the particular point in multiple consecutive inflation-deflation cycles;
calculate a difference between (a) an air pressure measured at the particular point in the current inflation-deflation cycle and (b) the rolling mean of the air pressure; and
increment an error counter if the difference between the measured air pressure and the rolling mean is greater than the rolling standard deviation;
wherein the error state is triggered if the error counter exceeds a predetermined number of counts.

5. The device (300) of any of claims 1-4, wherein the controller (306) is further programmed to:
based on the signal from the pressure sensor, determine the time taken to inflate the balloon pump (301) once;
compare the time required to inflate the balloon pump (301) to a target time;
if the time required to inflate the balloon is not within a predetermined range of the target time, calculate the power input to the external driver (305) necessary to cause the time required to inflate the balloon to match the target time; and
adjust the amount of power subsequently provided to the driver (305) based on the calculation.

6. The device (300) of any of claims 1-5 further comprising a sensor
wherein the sensor is connected to the controller (306) so as to allow transmission of a signal to the controller (306), the signal representative of a quantity detected by the sensor; and
wherein the controller (306) is programmed to determine based on the signal from the sensor whether to operate the driver (305) in the closed mode or the open mode.

7. The device (300) of claim 6, wherein the sensor is a humidity sensor, and the quantity detected by the sensor is a humidity of the pumping air.

8. The device (300) of any of claims 1-7 further comprising EKG sensor and a pressure sensor;
wherein the EKG sensor detects an EKG signal, and is so coupled to the controller (306) as to allow transmission of an EKG signal to the controller (306);
wherein the pressure sensor detects or infers a ventricular pressure and is so coupled to the controller (306) as to allow transmission of a pressure signal to the controller (306); and
wherein the controller (306) is programmed to detect a QRS complex based on the EKG signal;
detect a dicrotic notch based on the pressure signal;
trigger the driver to deflate the pump following the QRS complex and inflate the pump following the dicrotic notch.

9. The device (300) of any of claims 1-8 wherein the skin interface (303, 600) comprises:
two portions (601, 606) fixed to one another and rotatable with respect to one another; receptacles (602, 604, 607, 609) for receiving air lines (603, 608) and electrical lines (605, 610) on both portions (601, 606);
an air-tight conduit between the receptacles (602, 604) and running through the interface (303, 600), for transmitting air through the interface (303, 600);
a wireless electrical coupling between the receptacles (602, 604, 607, 609), for transmitting electrical signals and power through the interface (303, 600);
a memory in which patient-specific parameters are stored; and
a processor that receives electrocardiogram signals, digitizes them, and produces a digital output indicative of the electrocardiogram signals.

10. The device (300, 700) of any of claims 1-9 wherein:
the internal drive line (702) has a first end adapted to be connected to the skin interface device (703) and a second end forming an air tight connection to the balloon pump (701);
the internal drive line (302) has a extravascular region (706) adjacent to the first end, a pump region (704) adjacent to the second end, and an arterial region (705) between the extravascular region (706) and the pump region (704);
wherein the cross-sectional area of the arterial region (705) is less than 50% of the internal cross-sectional area of the artery (2) in which it is to be deployed; and
wherein the cross-sectional areas of the extravascular (706) and pump (704) regions are larger than the cross-sectional area of the arterial region (705) ; and
wherein the diameter of the pump region is sized and shaped to connect to the pump.

11. The device (700) of claim 10 wherein:
the extravascular region (706) has an inner diameter of about 6 mm;
the arterial region (705) has an outer diameter of about 5 mm and is adapted to be deployed in the subclavian artery; and
the pump region (704) has an outer diameter of about 6 mm.

12. The device (300, 700) of any of claims 1-11 wherein the balloon pump (301, 601, 701) comprises polyurethane modified with hydrophobic end groups.

13. The device (300, 700) of claim 12 wherein the hydrophobic end groups are silicone groups.

14. The device (300, 700) of any of claims 1-13 wherein:
the arterial interface (307) comprises a vascular graft (1), and a stopper (5);
the vascular graft (1) defines a graft lumen and comprises a distal end (4) so sized and shaped as to be suited for grafting to an artery (2);
the stopper (5) fills the graft lumen except for a hole (6) defined through the stopper's length, the hole (6) providing a conduit through the graft lumen;
the internal drive line (302, 605, 702) passes through the conduit and is immobilized relative to the stopper (5);
the graft lumen is wide enough to allow passage of the balloon pump chamber (8); and
the conduit is too narrow to allow passage of the balloon pump chamber (8).

15. The device of claim 14 wherein the stopper (5) is formed as two pieces that are clamped into contact with one another around the internal drive line (302, 605, 702) to immobilize the internal drive line (302, 605, 702) relative to the stopper (5).

## Patentansprüche

1. Ventrikuläres Assistenzgerät (300), umfassend:
eine intra-aortale Ballonpumpe (301), die so bemessen und geformt ist, dass sie in der Aorta eines Patienten hängt, wobei die Pumpe (301) eine Wand aufweist, die zumindest teilweise aus einem feuchtigkeitsbeständigen Material hergestellt ist;
eine Hautschnittstelle (303);
einen externen Antrieb (305), der einen starren Balg (401), einen Antriebsmotor (406) zum Betätigen des Balgs (401) und Ventile umfasst, die angeordnet sind, um mehrere Pumpmodi zu ermöglichen;
eine interne Antriebsleitung (302), die die Ballonpumpe (301) mit der Hautschnittstelle (303) verbindet;
eine arterielle Schnittstelle (307), die so bemessen und geformt ist, dass sie die interne Antriebsleitung (302) durch eine Arterienwand führt;
eine externe Antriebsleitung (304), die die Hautschnittstelle (303) mit dem Antrieb (305) verbindet; und
eine Steuerung (306);
wobei
die Ballonpumpe (301), die interne Antriebsleitung (302), die Hautschnittstelle (303), die externe Antriebsleitung (304) und der Antrieb (305) ein Fluidsystem zur Aufnahme eines Pumpmediums bilden; und
die Steuerung (306) programmiert ist zum:
Betätigen der Ventile (402, 403, 404) und des Antriebsmotors (406) derart, dass das Pumpmedium nicht mehr als einen vorbestimmten Betrag über einen erfassten Blutdruck hinaus unter Druck gesetzt wird;
Veranlassen der Ventile (402, 403, 404), einen geschlossenen Pumpmodus einzunehmen, wobei der Vorrichtung (300) kein Pumpmedium zugeführt oder aus ihr abgelassen wird;
Veranlassen des Antriebs (305), während des geschlossenen Modus durch mehrere aufeinanderfolgende Aufblas-/Ablasszyklen zu pumpen;
**dadurch gekennzeichnet, dass**:
das in dem Fluidsystem vorhandene Pumpmedium Umgebungsluft (405) umfasst, und die Steuerung (306) programmiert ist, die Ventile (402, 403, 404) zu veranlassen, einen offenen Modus einzunehmen, in dem das Pumpmedium aus dem Fluidsystem entlüftet und durch Umgebungsluft (405) ersetzt wird.

2. Vorrichtung (300) nach Anspruch 1, wobei in dem geschlossenen Pumpmodus die Ballonpumpe (301), die interne Antriebsleitung (302), die Hautschnittstelle (303), die externe Antriebsleitung (304) und der Antrieb (305) ein geschlossenes Fluidsystem bilden, das mit einer Pumpenluftsäule gefüllt ist, die als das Pumpmedium wirkt;
wobei die Vorrichtung (300) ferner einen Drucksensor umfasst, der den Luftdruck innerhalb des geschlossenen Fluidsystems erfasst, wobei der Drucksensor so mit der Steuerung (306) verbunden ist, dass er die Übertragung eines den Druck darstellenden Signals an die Steuerung (306) ermöglicht; und wobei die Steuerung (306) programmiert ist, das Signal von dem Drucksensor zu empfangen;
den Druck der Luftsäule mit einem vorbestimmten normalen Betriebsbereich durch einen vorbestimmten Satz von Betriebskriterien zu vergleichen; und
einen Fehlerzustand auszulösen, wenn der Druck außerhalb des normalen Betriebsbereichs liegt.

3. Vorrichtung (300) nach Anspruch 2, wobei die Steuerung (306) ferner programmiert ist zum:
Berechnen eines gleitenden Mittelwerts und einer gleitenden Standardabweichung des Luftdrucks der Pumpluft an einem bestimmten Punkt in den Aufblas-Ablass-Zyklen basierend auf Signalen von dem Luftdrucksensor an dem bestimmten Punkt in mehreren aufeinanderfolgenden Aufblas-Ablass-Zyklen; und
Berechnen einer Differenz zwischen (a) einem Luftdruck, der an dem bestimmten Punkt in dem aktuellen Aufblas-Ablass-Zyklus gemessen wurde, und (b) dem gleitenden Mittelwert des Luftdrucks;
wobei der Fehlerzustand ausgelöst wird, wenn die Differenz größer als das Doppelte der gleitenden Standardabweichung des Luftdrucks ist.

4. Vorrichtung (300) nach Anspruch 2 oder 3, wobei die Steuerung (306) ferner programmiert ist zum:
Berechnen eines gleitenden Mittelwerts und einer gleitenden Standardabweichung des Luftdrucks der Pumpluft an einem bestimmten Punkt in den Aufblas-Ablass-Zyklen basierend auf Signalen von dem Luftdrucksensor an dem bestimmten Punkt in mehreren aufeinanderfolgenden Aufblas-Ablass-Zyklen;
Berechnen einer Differenz zwischen (a) einem Luftdruck, der an dem bestimmten Punkt in dem aktuellen Aufblas-Ablass-Zyklus gemessen wurde, und (b) dem gleitenden Mittelwert des Luftdrucks; und
Inkrementieren eines Fehlerzählers, wenn die Differenz zwischen dem gemessenen Luftdruck und dem gleitenden Mittelwert größer als die gleitende Standardabweichung ist;
wobei der Fehlerzustand ausgelöst wird, wenn der Fehlerzähler eine vorbestimmte Anzahl von Zählungen überschreitet.

5. Vorrichtung (300) nach einem der Ansprüche 1-4, wobei die Steuerung (306) ferner programmiert ist zum:
Ermitteln der zum einmaligen Aufblasen der Ballonpumpe (301) benötigten Zeit basierend auf dem Signal des Drucksensors;
Vergleichen der zum Aufblasen der Ballonpumpe (301) benötigten Zeit mit einer Zielzeit;
wenn die zum Aufblasen des Ballons benötigte Zeit nicht innerhalb eines vorbestimmten Bereichs der Zielzeit liegt, Berechnen der Leistung, die dem externen Antrieb (305) zugeführt werden muss, damit die zum Aufblasen des Ballons benötigte Zeit der Zielzeit entspricht; und
Anpassen des Betrags an Leistung, die anschließend dem Antrieb (305) zugeführt wird, basierend auf der Berechnung.

6. Vorrichtung (300) nach einem der Ansprüche 1-5, ferner umfassend einen Sensor,
wobei der Sensor mit der Steuerung (306) verbunden ist, um so die Übertragung eines Signals an die Steuerung (306) zu ermöglichen, wobei das Signal eine von dem Sensor erfasste Größe darstellt; und
wobei die Steuerung (306) programmiert ist, basierend auf dem Signal von dem Sensor zu ermitteln, ob der Antrieb (305) in dem geschlossenen Modus oder dem offenen Modus betrieben werden soll.

7. Vorrichtung (300) nach Anspruch 6, wobei der Sensor ein Feuchtigkeitssensor ist und die von dem Sensor erfasste Größe eine Feuchtigkeit der Pumpluft ist.

8. Vorrichtung (300) nach einem der Ansprüche 1-7, ferner umfassend einen EKG-Sensor und einen Drucksensor;
wobei der EKG-Sensor ein EKG-Signal erfasst und so mit der Steuerung (306) gekoppelt ist, dass er die Übertragung eines EKG-Signals an die Steuerung (306) ermöglicht;
wobei der Drucksensor einen ventrikulären Druck erkennt oder ableitet und so mit der Steuerung (306) gekoppelt ist, dass er die Übertragung eines Drucksignals an die Steuerung (306) ermöglicht; und
wobei die Steuerung (306) programmiert ist, einen QRS-Komplex basierend auf dem EKG-Signal zu erkennen;
eine dikrotische Kerbe basierend auf dem Drucksignal zu erkennen;
den Antrieb auszulösen, um die Pumpe nach dem QRS-Komplex abzulassen und die Pumpe nach der dikrotischen Kerbe aufzublasen.

9. Vorrichtung (300) nach einem der Ansprüche 1-8, wobei die Hautschnittstelle (303, 600) umfasst:
zwei Abschnitte (601, 606), die aneinander befestigt und in Bezug aufeinander drehbar sind;
Behälter (602, 604, 607, 609) zur Aufnahme von Luftleitungen (603, 608) und elektrischen Leitungen (605, 610) an beiden Abschnitten (601, 606);
eine luftdichte Leitung zwischen den Behältern (602, 604), die durch die Schnittstelle (303, 600) verläuft, um Luft durch die Schnittstelle (303, 600) zu übertragen;
eine drahtlose elektrische Kopplung zwischen den Behältern (602, 604, 607, 609) zum Übertragen elektrischer Signale und Leistung über die Schnittstelle (303, 600);
einen Speicher, in dem patientenspezifische Parameter gespeichert sind; und
einen Prozessor, der Elektrokardiogrammsignale empfängt, digitalisiert und eine digitale Ausgabe erzeugt, die die Elektrokardiogrammsignale anzeigt.

10. Vorrichtung (300, 700) nach einem der Ansprüche 1-9, wobei:
die interne Antriebsleitung (702) ein erstes Ende aufweist, das zur Verbindung mit der Hautschnittstellenvorrichtung (703) angepasst ist, und ein zweites Ende, das eine luftdichte Verbindung mit der Ballonpumpe (701) bildet;
die interne Antriebsleitung (302) einen extravaskulären Bereich (706) angrenzend an das erste Ende, einen Pumpbereich (704) angrenzend an das zweite Ende, und einen arteriellen Bereich (705) zwischen dem extravaskulären Bereich (706) und dem Pumpbereich (704) aufweist;
wobei die Querschnittsfläche des arteriellen Bereichs (705) weniger als 50 % der inneren Querschnittsfläche der Arterie (2) beträgt, in der sie eingesetzt werden soll; und
wobei die Querschnittsflächen der extravaskulären (706) und des Pumpenbereichs (704) größer sind als die Querschnittsfläche des arteriellen Bereichs (705); und
wobei der Durchmesser des Pumpenbereichs so bemessen und geformt ist, dass er mit der Pumpe verbunden werden kann.

11. Vorrichtung (700) nach Anspruch 10, wobei:
der extravaskuläre Bereich (706) einen Innendurchmesser von etwa 6 mm aufweist;
der arterielle Bereich (705) einen Außendurchmesser von etwa 5 mm aufweist und zum Einsetzen in die Arteria subclavia geeignet ist; und
der Pumpenbereich (704) einen Außendurchmesser von etwa 6 mm aufweist.

12. Vorrichtung (300, 700) nach einem der Ansprüche 1-11, wobei die Ballonpumpe (301, 601, 701) Polyurethan umfasst, das mit hydrophoben Endgruppen modifiziert ist.

13. Vorrichtung (300, 700) nach Anspruch 12, wobei die hydrophoben Endgruppen Silikongruppen sind.

14. Vorrichtung (300, 700) nach einem der Ansprüche 1-13, wobei:
die arterielle Schnittstelle (307) ein vaskuläres Transplantat (1) und einen Stopper (5) umfasst;
das vaskuläre Transplantat (1) ein Transplantatlumen definiert und ein distales Ende (4) umfasst, das bemessen und geformt ist, um für das Transplantieren an eine Arterie (2) geeignet zu sein;
der Stopper (5) das Transplantatlumen ausfüllt, mit Ausnahme einer Öffnung (6), die durch die Länge des Stoppers definiert ist, wobei die Öffnung (6) eine Leitung durch das Transplantatlumen bereitstellt;
die interne Antriebsleitung (302, 605, 702) durch die Leitung verläuft und relativ zum Stopper (5) immobilisiert ist;
das Transplantatlumen ausreichend breit ist, um den Durchgang der Ballonpumpenkammer (8) zu ermöglichen; und
die Leitung zu schmal ist, um den Durchgang der Ballonpumpenkammer (8) zu ermöglichen.

15. Vorrichtung nach Anspruch 14, wobei der Stopper (5) als zwei Teile ausgebildet ist, die um die innere Antriebsleitung (302, 605, 702) herum in Kontakt miteinander geklemmt sind, um die innere Antriebsleitung (302, 605, 702) relativ zum Stopper (5) zu immobilisieren.

## Revendications

1. Dispositif d'assistance ventriculaire (300), comprenant :
une pompe à ballonnet intra-aortique (301) dimensionnée et conformée pour être suspendue à l'intérieur de l'aorte d'un patient, la pompe (301) ayant une paroi réalisée au moins en partie à partir d'un matériau résistant à l'humidité ;
une interface cutanée (303) ;
un dispositif d'entraînement externe (305) comprenant un soufflet rigide (401), un moteur principal (406) pour l'actionnement du soufflet (401), et des valves agencées pour permettre de multiples modes de pompage ;
une ligne d'entraînement interne (302) connectant la pompe à ballonnet (301) à l'interface cutanée (303) ;
une interface artérielle (307), dimensionnée et conformée pour faire passer la ligne d'entraînement interne (302) à travers une paroi artérielle ;
une ligne d'entraînement externe (304) connectant l'interface cutanée (303) au dispositif d'entraînement (305) ; et
un dispositif de commande (306) ;
dans lequel
la pompe à ballonnet (301), la ligne d'entraînement interne (302), l'interface cutanée (303), la ligne d'entraînement externe (304) et le dispositif d'entraînement (305) forment un système de fluide pour la contention d'un milieu de pompage ; et
le dispositif de commande (306) est programmé pour :
actionner les valves (402, 403, 404) et le moteur principal (406) de sorte que le milieu de pompage ne soit pas pressurisé plus qu'à une quantité prédéterminée au-dessus d'une pression sanguine détectée ;
amener les valves (402, 403, 404) à adopter un mode de pompage fermé dans lequel aucun milieu de pompage n'est ajouté au dispositif (300) ou évacué de celui-ci ;
amener le dispositif d'entrainement (305) à pomper à travers de multiples cycles consécutifs de gonflage-dégonflage durant le mode fermé ;
**caractérisé en ce que** :
le milieu de pompage présent dans le système de fluide comprend de l'air ambiant (405), et le dispositif de commande (306) est programmé pour amener les valves (402, 403, 404) à adopter un mode ouvert dans lequel le milieu de pompage est évacué du système de fluide et remplacé par de l'air ambiant (405).

2. Dispositif (300) selon la revendication 1, dans lequel, dans le mode de pompage fermé, la pompe à ballonnet (301), la ligne d'entraînement interne (302), l'interface cutanée (303), la ligne d'entraînement externe (304) et le dispositif d'entraînement (305) forment un système de fluide fermé chargé avec une colonne d'air de pompage qui agit comme le milieu de pompage ;
le dispositif (300) comprenant en outre un capteur de pression qui détecte la pression d'air à l'intérieur du système de fluide fermé, le capteur de pression étant connecté au dispositif de commande (306) de manière à permettre la transmission d'un signal représentatif de la pression au dispositif de commande (306) ; et dans lequel le dispositif de commande (306) est programmé pour recevoir le signal à partir du capteur de pression ;
comparer la pression de la colonne d'air à une plage de fonctionnement normal prédéterminée par un ensemble prédéterminé de critères de fonctionnement ; et
déclencher une condition d'erreur si la pression est en dehors de la plage de fonctionnement normal.

3. Dispositif (300) selon la revendication 2, dans lequel le dispositif de commande (306) est en outre programmé pour :
calculer une moyenne de roulement et un écart type de roulement de la pression d'air de l'air de pompage à un point particulier dans les cycles de gonflage-dégonflage sur base de signaux provenant du capteur de pression d'air sur le point particulier dans de multiples cycles consécutifs de gonflage-dégonflage ; et
calculer une différence entre (a) une pression d'air mesurée au point particulier dans le cycle de gonflage-dégonflage actuel et (b) la moyenne de roulement de la pression d'air ;
dans lequel l'état d'erreur est déclenché si la différence est supérieure à deux fois l'écart type de roulement de la pression d'air.

4. Dispositif (300) selon l'une quelconque des revendications 2 ou 3, dans lequel le dispositif de commande (306) est en outre programmé pour :
calculer une moyenne de roulement et un écart type de roulement de la pression d'air de l'air de pompage à un point particulier dans les cycles de gonflage-dégonflage sur base de signaux provenant du capteur de pression d'air au point particulier dans de multiples cycles consécutifs de gonflage-dégonflage ;
calculer une différence entre (a) une pression d'air mesurée au point particulier dans le cycle de gonflage-dégonflage actuel et (b) la moyenne de roulement de la pression d'air ; et
incrémenter un compteur d'erreurs si la différence entre la pression d'air mesurée et la moyenne de roulement est supérieure à l'écart type de roulement ;
dans lequel l'état d'erreur est déclenché si le compteur d'erreur dépasse un nombre prédéterminé de comptages.

5. Dispositif (300) selon l'une quelconque des revendications 1-4, dans lequel le dispositif de commande (306) est en outre programmé pour :
sur base du signal provenant du capteur de pression, déterminer le temps pris pour gonfler la pompe à ballonnet (301) une fois ;
comparer le temps requis pour gonfler la pompe à ballonnet (301) à un temps cible ;
si le temps requis pour gonfler le ballonnet n'est pas dans une plage prédéterminée du temps cible, calculer l'apport de puissance vers le dispositif d'entrainement externe (305) nécessaire pour amener le temps requis pour gonfler le ballonnet à correspondre au temps cible ; et
ajuster la quantité de puissance fournie ultérieurement au dispositif d'entrainement (305) sur base du calcul.

6. Dispositif (300) selon l'une quelconque des revendications 1-5, comprenant en outre un capteur
dans lequel le capteur est connecté au dispositif de commande (306) de manière à permettre la transmission d'un signal au dispositif de commande (306), le signal étant représentatif d'une quantité détectée par le capteur ; et
dans lequel le dispositif de commande (306) est programmé pour déterminer sur base du signal provenant du capteur s'il faut faire fonctionner le dispositif d'entrainement (305) dans le mode fermé ou le mode ouvert.

7. Dispositif (300) selon la revendication 6, dans lequel le capteur est un capteur d'humidité, et la quantité détectée par le capteur est une humidité de l'air de pompage.

8. Dispositif (300) selon l'une quelconque des revendications 1-7, comprenant en outre un capteur ECG et un capteur de pression ;
dans lequel le capteur ECG détecte un signal ECG, et est couplé au dispositif de commande (306) de manière à permettre la transmission d'un signal ECG au dispositif de commande (306) ;
dans lequel le capteur de pression détecte ou déduit une pression ventriculaire et est couplé au dispositif de commande (306) de manière à permettre la transmission d'un signal de pression au dispositif de commande (306) ; et
dans lequel le dispositif de commande (306) est programmé pour détecter un complexe QRS sur base du signal ECG ;
détecter une encoche dicrote sur base du signal de pression ;
à déclencher le dispositif d'entrainement pour dégonfler la pompe à la suite du complexe QRS et gonfler la pompe à la suite de l'encoche dicrote.

9. Dispositif (300) selon l'une quelconque des revendications 1-8, dans lequel l'interface cutanée (303, 600) comprend :
deux parties (601, 606) fixées l'une à l'autre et pouvant tourner l'une par rapport à l'autre ;
des réceptacles (602, 604, 607, 609) pour la réception des lignes d'air (603, 608) et des lignes électriques (605, 610) sur les deux parties (601, 606) ;
un conduit étanche à l'air entre les réceptacles (602, 604) et circulant à travers l'interface (303, 600), pour la transmission d'air à travers l'interface (303, 600) ;
un couplage électrique sans fil entre les réceptacles (602, 604, 607, 609), pour la transmission de signaux électriques et d'énergie à travers l'interface (303, 600) ;
une mémoire dans laquelle des paramètres spécifiques du patient sont stockés ; et
un processeur qui reçoit des signaux d'électrocardiogramme, les numérise, et produit une sortie numérique indicative des signaux d'électrocardiogramme.

10. Dispositif (300, 700) selon l'une quelconque des revendications 1-9 dans lequel :
la ligne d'entraînement interne (702) a une première extrémité adaptée pour être connectée au dispositif d'interface cutanée (703) et une deuxième extrémité formant une connexion étanche à l'air à la pompe à ballonnet (701) ;
la ligne d'entraînement interne (302) a une région extravasculaire (706) adjacente à la première extrémité, une région de pompage (704) adjacente à la deuxième extrémité, et une région artérielle (705) entre la région extravasculaire (706) et la région de pompage (704) ;
dans lequel la zone de section transversale de la région artérielle (705) est inférieure à 50% de la zone de section transversale interne de l'artère (2) dans laquelle elle doit être déployée ; et
dans lequel les zones de section transversale des régions extravasculaire (706) et de pompe (704) sont plus grandes que la zone de section transversale de la région artérielle (705) ; et
dans lequel le diamètre de la région de la pompe est dimensionné et conformé pour se connecter à la pompe.

11. Dispositif (700) selon la revendication 10, dans lequel :
la région extravasculaire (706) a un diamètre interne d'environ 6 mm ;
la région artérielle (705) a un diamètre externe d'environ 5 mm et est adaptée pour être déployée dans l'artère sous-clavière ; et
la région de pompe (704) a un diamètre externe d'environ 6 mm.

12. Dispositif (300, 700) selon l'une quelconque des revendications 1-11, dans lequel la pompe à ballonnet (301, 601, 701) comprend du polyuréthane modifié avec des groupes terminaux hydrophobes.

13. Dispositif (300, 700) selon la revendication 12, dans lequel les groupes terminaux hydrophobes sont des groupes silicone.

14. Dispositif (300, 700) selon l'une quelconque des revendications 1-13, dans lequel :
l'interface artérielle (307) comprend une greffe vasculaire (1), et une butée (5) ;
la greffe vasculaire (1) définit une lumière de greffe et comprend une extrémité distale (4) dimensionnée et conformée de manière à être adaptée pour une greffe sur une artère (2) ;
la butée (5) remplit la lumière de greffe à l'exception d'un trou (6) défini à travers la longueur de la butée, le trou (6) fournissant un conduit à travers la lumière de greffe ;
la ligne d'entraînement interne (302, 605, 702) passe à travers le conduit et est immobilisée par rapport à la butée (5) ;
la lumière de greffe est suffisamment large pour permettre le passage de la chambre de pompe à ballonnet (8) ; et
le conduit est trop étroit pour permettre le passage de la chambre de pompe à ballonnet (8).

15. Dispositif selon la revendication 14, dans lequel la butée (5) est formée de deux pièces qui sont serrées en contact l'une avec l'autre autour de la ligne d'entraînement interne (302, 605, 702) pour immobiliser la ligne d'entraînement interne (302, 605, 702) par rapport à la butée (5) .
